# EUROPEAN PATENT APPLICATION

(11) **EP 4 227 664 A1**
(43) Date of publication of application: **16.08.2023**
(21) Application number: 21879762.9
(22) Date of filing: 30.08.2021
(51) Int. Cl.: G01N 15/14

(54) **BIOPARTICLE SORTING DEVICE, BIOPARTICLE SORTING SYSTEM, AND INFORMATION PROCESSING DEVICE**

(30) Priority: 12.10.2020 US 202063090676 P
(71) Applicant: Sony Group Corporation, Tokyo 108-0075 (JP)
(72) Inventor: SUKIGARA, Ryu, Tokyo 108-0075 (JP); SATO, Koichiro, Tokyo 108-0075 (JP); OSAWA, Muneaki, Tokyo 108-0075 (JP); YOSHIDA, Shinichi, Tokyo 108-0075 (JP); YANASHITA, Yudai, Tokyo 108-0075 (JP); BRUN, Marcaurele, Tokyo 108-0075 (JP); MATSUDA, Hazime, Tokyo 108-0075 (JP)
(74) Representative: D Young & Co LLP
(86) International application number: PCT/JP2021/031725
(87) International publication number: WO 2022/080020

(57) **Abstract**

A main object of the present disclosure is to provide a biological particle sorting device that is easy to use or operate for both of two different types of users.

The present disclosure provides a biological particle sorting device that operates in a first display mode or a second display mode on the basis of identification information, the device including a first display mode for receiving an input of operation control data related to biological particle sorting processing and a second display mode in which a processing execution operation screen is displayed. In the first display mode, processing condition setting screen related to the biological particle sorting processing is displayed, and the biological particle sorting device may receive an input of the operation control data via the processing condition setting screen.

## Description

### TECHNICAL FIELD

The present disclosure relates to a biological particle sorting device, a biological particle sorting system, and an information processing device. More specifically, the present disclosure relates to a biological particle sorting device, a biological particle sorting system, and an information processing device that display a screen related to control and/or execution of biological particle sorting processing.

### BACKGROUND ART

In flow cytometry, light derived from various fluorescent dyes is analyzed multidimensionally. For the analysis, a large number of graphs and/or tables of statistical values are displayed on the screen of the information processing device, and for example, the gate is set or adjusted. Furthermore, desired biological particles are sorted using the result of the analysis. The device that executes the sorting is also called a cell sorter. Furthermore, in recent years, a device for sorting biological particles in a closed space has also been proposed, and the device is also called a closed sorter.

Several proposals have been made so far for closed sorters. For example, Patent Document 1 below discloses a microparticle sorting device including a determination unit that determines whether microparticles are sorted, on the basis of light generated by irradiating, with light, the microparticles flowing in a flow channel, in which the determination unit performs a primary sorting determination of determining, on the basis of characteristics of the light, whether the microparticles belong to any one of two or more different microparticle populations, and then performs a secondary sorting determination of determining, on the basis of a particle structure ratio specified for the two or more different microparticle populations, whether microparticles determined to belong to any one of the microparticle populations in the primary sorting determination are sorted.

### CITATION LIST

### PATENT DOCUMENT

Patent Document 1: JP 2020-076736 A

### SUMMARY OF THE INVENTION

### PROBLEMS TO BE SOLVED BY THE INVENTION

For the sorting processing by a biological particle sorting device, a sorting processing condition such as gate setting is set. The setting is performed via various pieces of data displayed on the screen as described above. In order to perform this setting, highly specialized knowledge (for example, knowledge regarding biological particles and knowledge regarding a sorting device) is often required. In devices such as a flow cytometer and a cell sorter which have been commercially available so far, a user interface thereof is designed on the assumption that a user has such specialized knowledge.

However, in order to sort a large amount of desired cells using a biological particle sorting device, for example, for producing a cell therapeutic agent, it is assumed that setting of the sorting processing conditions is performed by a user who is accustomed to using the various data (for example, a researcher or a developer having the highly specialized scientific knowledge), while the actual sorting operation is performed by a user who is not accustomed to using the various data (for example, a worker or an operator in a cell therapeutic agent producing factory) as compared with the device user.

Therefore, a main object of the present disclosure is to provide a biological particle sorting device that is easy to use or operate for both of two such types of users.

### SOLUTIONS TO PROBLEMS

The present disclosure provides
a biological particle sorting device that operates in a first display mode or a second display mode on the basis of identification information, the device including
a first display mode for receiving an input of operation control data related to biological particle sorting processing and
a second display mode in which a processing execution operation screen is displayed.

Furthermore, the present disclosure provides
an information processing device for controlling biological particle sorting processing
that is designed to receive input of operation control data related to biological particle sorting processing via a processing condition setting screen related to the biological particle sorting processing, and transmit the operation control data to a biological particle sorting device that executes the sorting processing or an information processing device that operates the sorting device, in which
the operation control data includes processing condition data adopted by the biological particle sorting device.

Furthermore, the present disclosure provides
an information processing device for executing biological particle sorting processing
that is designed to receive operation control data related to biological particle sorting processing and display a processing execution operation screen generated on the basis of the operation control data, and
cause the biological particle sorting device to execute sorting processing according to an instruction input by an operator via the processing execution operation screen, in which
the operation control data includes gate information for specifying biological particles sorted in the processing, and
adjustment of at least a part of the gate information is restricted in the processing execution operation screen.

Furthermore, the present disclosure provides
a biological particle sorting system including
a first biological particle sorting device or information processing device that operates in a first display mode for receiving an input of operation control data related to biological particle sorting processing; and
a second biological particle sorting device or information processing device that operates in a second display mode in which a processing execution operation screen is displayed.

### BRIEF DESCRIPTION OF DRAWINGS

Fig. 1 is a schematic diagram showing an example of a situation in which a biological particle sorting device is operated.
Fig. 2A is a diagram showing an example configuration of a biological sample analyzer of the present disclosure.
Fig. 2B is an example of a block diagram of the biological particle sorting device of the present disclosure.
Fig. 3 is an example of a flowchart of processing performed by the biological particle sorting device of the present disclosure.
Fig. 4 is a diagram showing an example of a mode selection screen.
Fig. 5 is a diagram showing an example of an identification information input screen.
Fig. 6 is a diagram showing an example of a processing condition setting screen.
Fig. 7 is a diagram showing an example of the processing condition setting screen.
Fig. 8 is a diagram showing an example of a first display area.
Fig. 9 is a diagram showing an example of a second display area.
Fig. 10A is a diagram for explaining a tab area.
Fig. 10B is a diagram for explaining the tab area.
Fig. 10C is a diagram for explaining the tab area.
Fig. 11 is a diagram showing an example of a processing flow in a first display mode.
Fig. 12 is a diagram showing an example of the processing condition setting screen.
Fig. 13 is a diagram showing an example of the processing condition setting screen.
Fig. 14 is a diagram showing an example of a setting information display area.
Fig. 15A is a diagram showing an example of a window displayed by selecting a matrix display button.
Fig. 15B is a diagram showing an example of a state of adjustment of each correction coefficient in a correction matrix.
Fig. 16 is a diagram showing an example of the window displayed by selecting a gate tree display button.
Fig. 17 is a diagram showing an example of a toolbox displayed by selecting a worksheet toolbox display button.
Fig. 18 is a diagram showing an example of the toolbox displayed by selecting a plot toolbox display button.
Fig. 19 is a diagram showing an example of the toolbox displayed by selecting a gate toolbox display button.
Fig. 20 is a diagram showing an example of a screen for controlling sorting processing.
Fig. 21 is a diagram showing an example of a template property area.
Fig. 22 is a diagram showing an example of a screen of an aliquot setting area.
Fig. 23 is a diagram showing an example of an operator-oriented instruction data input area.
Fig. 24 is a diagram showing an example of the processing execution operation screen.
Fig. 25 is a diagram showing an example of a fourth display area.
Fig. 26 is a diagram showing an example of the processing execution operation screen.
Fig. 27 is a diagram showing an example of the processing execution operation screen.
Fig. 28A is a diagram showing an example of the processing execution operation screen.
Fig. 28B is a diagram showing an example of the processing execution operation screen.
Fig. 29 is a diagram showing an example of the processing execution operation screen.
Fig. 30 is a diagram showing an example of a window displayed at the time of acquiring an aliquot.
Fig. 31 is a diagram showing an example of a biological particle sorting system of the present disclosure.
Fig. 32A is a diagram showing an example of an operation panel.
Fig. 32B is a diagram showing an example of the operation panel.
Fig. 33A is a diagram showing an example of a slider bar.
Fig. 33B is a diagram showing an example of the slider bar.
Fig. 33C is a diagram showing an example of the slider bar.
Fig. 33D is a diagram showing an example of the slider bar.
Fig. 34A is a diagram for explaining an enlarged image displayed in gate adjustment.
Fig. 34B is a diagram for explaining an enlarged image displayed in the gate adjustment.

### MODE FOR CARRYING OUT THE INVENTION

Preferred embodiments for carrying out the present disclosure will be described below. Note that the embodiments described below show representative embodiments of the present disclosure, and the scope of the present disclosure is not limited to these embodiments. Note that the present disclosure will be described in the following order.
1. Basic Concept of the Present Disclosure
2. First Embodiment of the Present Disclosure (Biological Particle Sorting Device)
   (1) Device Example Configuration
   (2) Example of Processing
      (2-1) Selection of Display Mode
      (2-2) First Display Mode
      (2-2-1) Example Configuration of Screen Displayed in First Display Mode
      (2-2-2) Example of Processing in First Display Mode
      (2-3) Second Display Mode
      (2-3-1) Example Configuration of Screen Displayed in Second Display Mode
      (2-3-2) Example of Processing in Second Display Mode
   (3) Modified Example
3. Second Embodiment of the Present Disclosure (Biological Particle Sorting System)

### 1. Basic Concept of the Present Disclosure

As described above, the sorting processing condition setting for the sorting processing by the biological particle sorting device is performed via various data displayed on the screen, and highly specialized knowledge is often required to set the sorting processing condition. Therefore, it is considered difficult for a user such as the worker or the operator described above to appropriately use the conventional user interface. Therefore, it is desirable to provide a user interface that is easy for a user to use or operate.

It is also conceivable that a worker or an operator operates the biological particle sorting device while referring to a work procedure manual created by a researcher or a developer. However, for example, a biological particle-containing sample such as a cellcontaining sample has a different particle composition for each sample, and it is required to adjust the sorting processing condition for each sample. Furthermore, creating the work procedure manual is troublesome for the researcher or the developer, and referring to the work procedure manual is also a burden on the worker or the operator. Moreover, in an environment such as a clean room, since a worker or an operator wears clean room wear or lab gloves, it is often considered difficult to perform work while viewing an operation procedure manual.

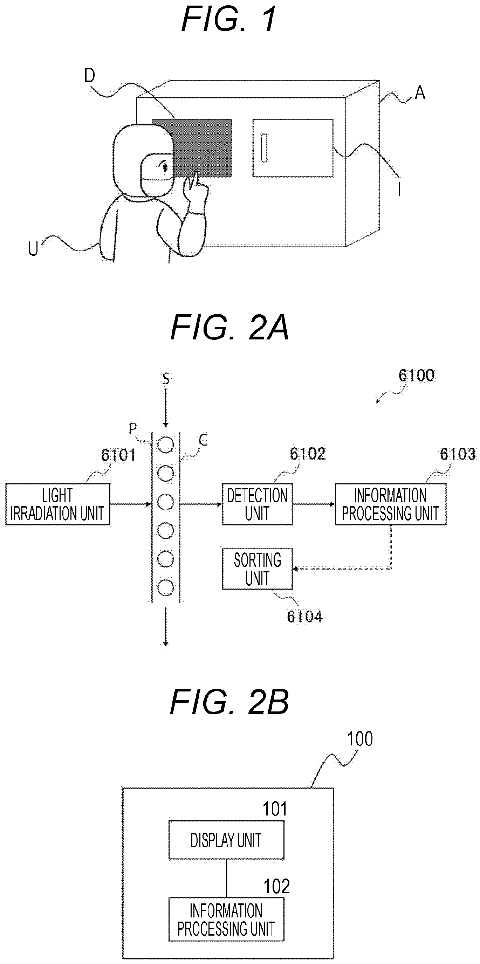

Furthermore, for example, research, development, and manufacturing related to a cell therapeutic agent are performed in a clean room in order to avoid contamination. Therefore, for example, in a case where the biological particle sorting device such as a closed sorter is used in a clean room, for example, as shown in Fig. 1, it is assumed that a user U operates a biological particle sorting device A while wearing work clothes and gloves for a clean room. The gloves are, for example, lab gloves including a material such as latex. In a case where such gloves are worn on the hands, fine manipulation is often difficult. For example, regarding the touch panel, it is difficult to perform fine operation while wearing the gloves. Therefore, it is desirable to provide a user interface that is easy to use or operate even in such a state.

The present disclosure provides a biological particle sorting device including a first display mode for receiving an input of operation control data related to biological particle sorting processing and a second display mode for displaying a processing execution operation screen. In an embodiment of the present disclosure, the biological particle sorting device may be designed to operate in the first display mode or the second display mode on the basis of identification information.

Since the biological particle sorting device according to the present disclosure has two display modes as described above, for example, a researcher or a developer can input operation control data using the first display mode, and then a worker or an operator can execute sorting processing with the biological particle sorting device via a processing execution operation screen in the second mode. Therefore, it is possible to present different user interfaces to the researcher or the developer and the worker or the operator, and for example, it is possible to provide a working environment suitable for each use.

For example, in the first display mode, the researcher or the developer inputs the operation control data (for example, work procedure information) related to the sorting processing operation to be executed by the worker or the operator via the processing condition setting screen. Then, in the second display mode, a processing execution operation screen generated on the basis of the operation control data is presented to the operator. Therefore, the researcher or the developer can easily create an instruction for the operator such as a correct operation procedure and/or a cautionary note for each type of sorting processing. Then, the operator can execute appropriate sorting processing according to the designated work procedure and referring to the cautionary notes according to the instruction displayed on the processing execution operation screen.

In the present specification, a user who operates the biological particle sorting device in the first display mode is also referred to as a "first user", and a user who operates the biological particle sorting device in the second display mode is also referred to as a "second user".

The first user may be a user who designs and/or develops the sorting processing to be executed in the second display mode. The first user is, for example, the researcher or developer described above.

The second user may be a user who operates the biological particle sorting device according to the instruction input in the first display mode to execute the sorting processing. The second user is, for example, the operator or the worker described above.

Hereinafter, first, an example configuration of the device will be described, and next, an example of the operation of the biological particle sorting device according to the present disclosure will be described.

### 2. First Embodiment of the Present Disclosure (Biological Particle Sorting Device)

### (1) Example Configuration of Device

The biological particle sorting device according to the present disclosure may be designed as a biological sample analyzer as will be described below.

Fig. 2A shows an example configuration of a biological sample analyzer of the present disclosure. A biological sample analyzer 6100 shown in Fig. 2A includes: a light irradiation unit 6101 that irradiates a biological sample S flowing in a flow channel C with light; a detection unit 6102 that detects light generated by irradiating the biological sample S with light; and an information processing unit 6103 that processes information about the light detected by the detection unit. The biological sample analyzer 6100 is a flow cytometer or an imaging cytometer, for example. The biological sample analyzer 6100 may include a sorting unit 6104 that sorts out specific biological particles P in a biological sample. The biological sample analyzer 6100 including the sorting unit is a cell sorter, for example.

### (Biological Sample)

The biological sample S may be a liquid sample containing biological particles. The biological particles are cells or non-cellular biological particles, for example. The cells may be living cells, and more specific examples thereof include blood cells such as erythrocytes and leukocytes, and germ cells such as sperms and fertilized eggs. Also, the cells may be those directly collected from a sample such as whole blood, or may be cultured cells obtained after culturing. The non-cellular biological particles are extracellular vesicles, or particularly, exosomes and microvesicles, for example. The biological particles may be labeled with one or more labeling substances (such as a dye (particularly, a fluorescent dye) and a fluorochrome-labeled antibody). Note that particles other than biological particles may be analyzed by the biological sample analyzer of the present disclosure, and beads or the like may be analyzed for calibration or the like.

### (Flow Channel)

The flow channel C is designed so that a flow of the biological sample S is formed. In particular, the flow channel C may be designed so that a flow in which the biological particles contained in the biological sample are aligned substantially in one row is formed. The flow channel structure including the flow channel C may be designed so that a laminar flow is formed. In particular, the flow channel structure is designed so that a laminar flow in which the flow of the biological sample (a sample flow) is surrounded by the flow of a sheath liquid is formed. The design of the flow channel structure may be appropriately selected by a person skilled in the art, or a known one may be adopted. The flow channel C may be formed in a flow channel structure such as a microchip (a chip having a flow channel on the order of micrometers) or a flow cell. The width of the flow channel C is 1 mm or smaller, or particularly, may be not smaller than 10 um and not greater than 1 mm. The flow channel C and the flow channel structure including the flow channel C may be made of a material such as plastic or glass.

The biological sample analyzer of the present disclosure is designed so that the biological sample flowing in the flow channel C, or particularly, the biological particles in the biological sample are irradiated with light from the light irradiation unit 6101. The biological sample analyzer of the present disclosure may be designed so that the irradiation point of light on the biological sample is located in the flow channel structure in which the flow channel C is formed, or may be designed so that the irradiation point of light is located outside the flow channel structure. An example of the former case may be a configuration in which the light is emitted onto the flow channel C in a microchip or a flow cell. In the latter case, the biological particles after exiting the flow channel structure (particularly, the nozzle portion thereof) may be irradiated with the light, and a flow cytometer of a jet-in-air type can be adopted, for example.

### (Light Irradiation Unit)

The light irradiation unit 6101 includes a light source unit that emits light, and a light guide optical system that guides the light to the irradiation point. The light source unit includes one or more light sources. The type of the light source(s) is a laser light source or an LED, for example. The wavelength of light to be emitted from each light source may be any wavelength of ultraviolet light, visible light, and infrared light. The light guide optical system includes optical components such as beam splitters, mirrors, or optical fibers, for example. Furthermore, the light guide optical system may also include a lens group for condensing light, and includes an objective lens, for example. There may be one or more irradiation points at which the biological sample and light intersect. The light irradiation unit 6101 may be designed to collect light emitted onto one irradiation point from one light source or different light sources.

### (Detection Unit)

The detection unit 6102 includes at least one photodetector that detects light generated by emitting light onto biological particles. The light to be detected may be fluorescence or scattered light (such as one or more of the following: forward scattered light, backscattered light, and side scattered light), for example. Each photodetector includes one or more light receiving elements, and has a light receiving element array, for example. Each photodetector may include one or more photomultiplier tubes (PMTs) and/or photodiodes such as APDs and MPPCs, as the light receiving elements. Each photodetector includes a PMT array in which a plurality of PMTs is arranged in a one-dimensional direction, for example. The detection unit 6102 may also include an image sensor such as a CCD or a CMOS. With the image sensor, the detection unit 6102 can acquire an image (such as a bright-field image, a dark-field image, or a fluorescent image, for example) of biological particles.

The detection unit 6102 includes a detection optical system that causes light of a predetermined detection wavelength to reach the corresponding photodetector. The detection optical system includes a spectroscopic unit such as a prism or a diffraction grating, or a wavelength separation unit such as a dichroic mirror or an optical filter. The detection optical system is designed to disperse the light generated by light irradiation to biological particles, for example, and detect the dispersed light with a larger number of photodetectors than the number of fluorescent dyes with which the biological particles are labeled. A flow cytometer including such a detection optical system is called a spectral flow cytometer. Further, the detection optical system is designed to separate the light corresponding to the fluorescence wavelength band of a specific fluorescent dye from the light generated by the light irradiation to the biological particles, for example, and cause the corresponding photodetector to detect the separated light.

The detection unit 6102 may also include a signal processing unit that converts an electrical signal obtained by a photodetector into a digital signal. The signal processing unit may include an A/D converter as a device that performs the conversion. The digital signal obtained by the conversion performed by the signal processing unit can be transmitted to the information processing unit 6103. The digital signal can be handled as data related to light (hereinafter, also referred to as "light data") by the information processing unit 6103. The light data may be light data including fluorescence data, for example. More specifically, the light data may be data of light intensity, and the light intensity may be light intensity data of light including fluorescence (the light intensity data may include feature quantities such as area, height, and width).

### (Information Processing Unit)

The information processing unit 6103 includes a processing unit that performs processing of various kinds of data (light data, for example), and a storage unit that stores various kinds of data, for example. In a case where the processing unit acquires the light data corresponding to a fluorescent dye from the detection unit 6102, the processing unit can perform fluorescence leakage correction (a compensation process) on the light intensity data. In the case of a spectral flow cytometer, the processing unit also performs a fluorescence separation process on the light data, and acquires the light intensity data corresponding to the fluorescent dye. The fluorescence separation process may be performed by an unmixing method disclosed in JP 2011-232259 A, for example. In a case where the detection unit 6102 includes an image sensor, the processing unit may acquire morphological information about the biological particles, on the basis of an image acquired by the image sensor. The storage unit may be designed to be capable of storing the acquired light data. The storage unit may be designed to be capable of further storing spectral reference data to be used in the unmixing processing.

In a case where the biological sample analyzer 6100 includes the sorting unit 6104 described later, the information processing unit 6103 can determine whether to sort the biological particles, on the basis of the light data and/or the morphological information. The information processing unit 6103 then controls the sorting unit 6104 on the basis of the result of the determination, and the biological particles can be sorted by the sorting unit 6104.

The information processing unit 6103 may be designed to be capable of outputting various kinds of data (such as light data and images, for example). For example, the information processing unit 6103 can output various kinds of data (such as a two-dimensional plot or a spectrum plot, for example) generated on the basis of the light data. The information processing unit 6103 may also be designed to be capable of accepting inputs of various kinds of data, and accepts a gating process on a plot by a user, for example. The information processing unit 6103 may include an output unit (such as a display, for example) or an input unit (such as a keyboard, for example) for performing the output or the input.

The information processing unit 6103 may be designed as a general-purpose computer, and may be designed as an information processing device that includes a CPU, a RAM, and a ROM, for example. The information processing unit 6103 may be included in the housing in which the light irradiation unit 6101 and the detection unit 6102 are included, or may be located outside the housing. Further, the various processes or functions to be executed by the information processing unit 6103 may be realized by a server computer or a cloud connected via a network.

### (Sorting Unit)

The sorting unit 6104 performs sorting of biological particles, in accordance with the result of determination performed by the information processing unit 6103. The sorting method may be a method by which droplets containing biological particles are generated by vibration, electric charges are applied to the droplets to be sorted, and the traveling direction of the droplets is controlled by an electrode. The sorting method may be a method for sorting by controlling the traveling direction of biological particles in the flow channel structure. The flow channel structure has a control mechanism based on pressure (injection or suction) or electric charge, for example. An example of the flow channel structure may be a chip (the chip disclosed in JP 2020-76736 A, for example) that has a flow channel structure in which the flow channel C branches into a recovery flow channel and a waste liquid flow channel on the downstream side, and specific biological particles are collected in the recovery flow channel.

### (2) Example of Processing

The biological particle sorting device according to the present disclosure may have a display unit D capable of touch input, for example, as shown in Fig. 1. As shown in the drawing, the display unit may be arranged on any surface of the housing of the device, particularly on a side surface, and for example, the screen of the display unit may be arranged at a position where a user standing (or sitting) near the device can perform touch input. A processing condition setting screen or a processing execution operation screen to be described later may be displayed on the display unit. Furthermore, as shown in the drawing, the biological particle sorting device according to the present disclosure may have an introduction section I for introducing a biological particle-containing sample into the device. For example, a container containing the sample is put into the device from the introduction unit, and the container is connected to a predetermined sample line. Then, after the connection, various processes that will be described below may be executed.

Note that, in the present specification, the touch operation may be a tap (single tap), a long tap, a double tap, or the like, but is not limited thereto. The touch operation may be, for example, dragging, swiping, pinching in, or pinching out. The type of the touch operation may be appropriately selected by those skilled in the art.

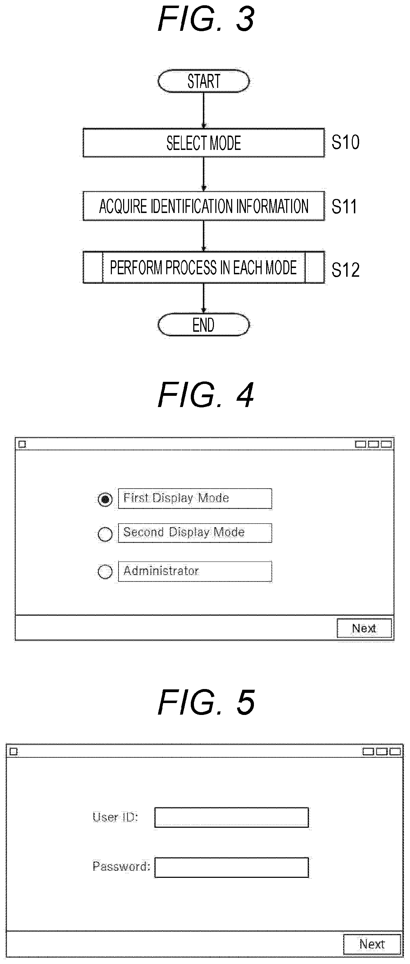

An example of processing executed by the biological particle sorting device according to the present disclosure will be described below with reference to Figs. 2B and 3. Fig. 2B is an example of a block diagram of the biological particle sorting device. As shown in the drawing, a biological particle sorting device 100 according to the present disclosure includes a display unit 101 and an information processing unit 102. Fig. 3 is an example of a flowchart of processing performed by the biological particle sorting device.

The display unit 101 outputs a screen in accordance with the present disclosure. The screen may be a screen based on data transmitted from the information processing unit 102. The display unit 101 may include a display device known in the art. For example, the display unit 101 includes a display device having a rectangular screen, in particular, a rectangle shape screen, and more particularly, a horizontally long rectangle shape screen. The size of the screen may be, for example, 10 inches or more, preferably 11 inches or more, and more preferably 12 inches or more. The upper limit of the size of the screen need not particularly be set, but may be, for example, 30 inches or less, 25 inches or less, 22 inches or less, or 20 inches or less. Such a screen size is suitable for display of a screen displayed in each mode described below.

The information processing unit 102 causes the display unit 101 to display a screen in accordance with the present disclosure. The information processing unit 102 is designed to output data for displaying the screen. The information processing unit 102 may be the information processing unit 6103 in (1) described above, but may be an information processing unit prepared separately. In addition, the biological particle sorting device 100 may include the light irradiation unit 6101, the detection unit 6102, and the sorting unit 6104 in (1) described above in addition to the display unit 101 and the information processing unit 102.

In the following (2-1) to (2-3), screens displayed by the biological particle sorting device 100 according to the present disclosure will be described.

### (2-1) Selection of Display Mode

In step S10, the biological particle sorting device 100 (particularly, the information processing unit 102) starts the processing.

In step S11, the information processing unit 102 causes the display unit 101 to output a mode selection screen that prompts the user to select a mode. For example, as shown in Fig. 4, the mode selection screen includes a button (a first display mode button) for selecting a first display mode and a button (a second display mode button) for selecting a second display mode. The mode selection screen may further include a button (a Next button) for displaying a next processing screen after the mode is selected.

A more specific mode name may be displayed on each button displayed on the mode selection screen. For example, as the first display mode, the sorting processing condition setting mode may be displayed, or a setting mode, a process development mode, a PD mode, or the like may be displayed. Furthermore, as the second display mode, a processing execution operation mode may be displayed, or an operating mode, an operation mode, an OP mode, or the like may be displayed.

As shown in the drawing, the mode selection screen may further display a button (an Administrator button) for selecting an administrator mode. In the administrator mode, the information processing unit 102 can set or change authority information of the user, for example.

In step S12, the information processing unit 102 causes the display unit 101 to output a screen prompting the input of the identification information of the user. The identification information may include a user ID (or a user name) and may further include a password. For example, as shown in Fig. 5, the screen includes a user ID input field and a password input field. The user inputs the identification information in these fields.

The information processing unit 102 refers to the input identification information and determines whether the operation of the device in the selected mode is permitted for the identification information. In a case where it is permitted, the information processing unit 102 advances the processing to step S13. In a case where it is not permitted, for example, the information processing unit 102 causes the display unit 101 to display a screen indicating that it is not permitted, and returns the processing to step S11 or S12.

In step S13, the processing in the selected mode is executed. The processing in each mode will be described in (2-2) and (2-3) below.

In the present disclosure, steps S12 and S13 may be omitted. In this case, selection of any mode in step S11 may be used as the identification information. Alternatively, only step S12 may be executed without executing step S11. That is, the information processing unit 102 may cause the display unit 101 to display a screen for receiving the input of the identification information, and then shift to the first display mode or the second display mode according to the input identification information.

### (2-2) First Display Mode

In the first display mode, the biological particle sorting device 100 (particularly, the information processing unit 102) receives an input of operation control data related to the biological particle sorting processing. In order to receive the input of the operation control data, the display unit 101 may be capable of touch input. The display unit 101 displays a processing condition setting screen that receives an input of operation control data related to the sorting processing of the biological particle-containing sample. The operation control data may be data used for executing the sorting processing in the second display mode, and in particular, includes data for generating a processing execution operation screen in the second display mode.

In the first display mode, the information processing unit 102 causes the display unit 101 to display a processing condition setting screen related to the biological particle sorting processing. The information processing unit 102 receives an input of the operation control data via the processing condition setting screen.

Note that the processing condition setting screen may be displayed on another display device instead of the display unit 101 provided in the biological particle sorting device 100. For example, the biological particle sorting device 100 may display the screen on a display device or an information processing device connected to the biological particle sorting device 100 in a wired or wireless manner via a network.

### (2-2-1) Example Configuration of Screen Displayed in First Display Mode

### (Processing Condition Setting Screen)

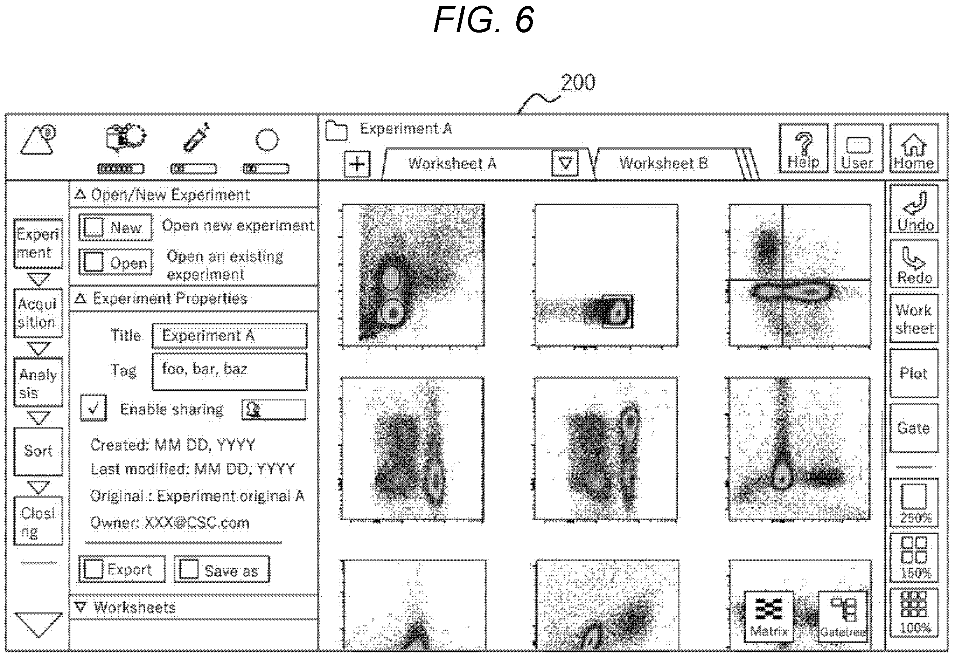

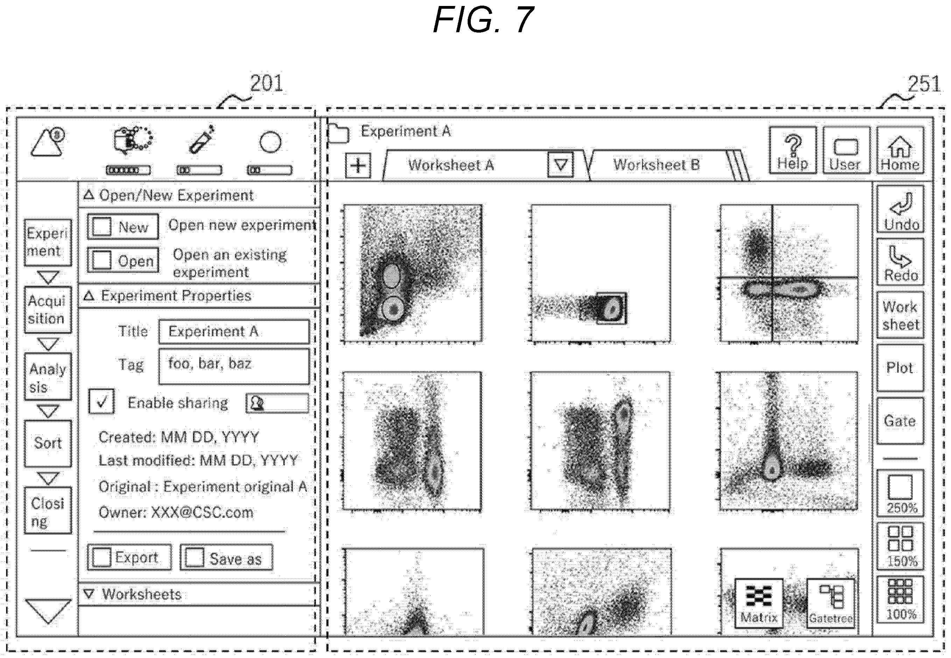

Fig. 6 shows an example configuration of the processing condition setting screen displayed on the display unit 101 by the biological particle sorting device 100 (particularly, the information processing unit 102). As shown in the drawing, the processing condition setting screen 200 shown in Fig. 7 includes a setting operation flow display area (hereinafter, also referred to as a "first display area") 201 for displaying an operation flow for setting the processing condition of the sorting processing, and a measurement data display area (hereinafter also referred to as a "second display area") 251 for displaying measurement data related to the biological particle-containing sample.

As shown in the drawing, the first display area may be arranged on the left side of the processing condition setting screen, and the second display area may be arranged on the right side of the processing condition setting screen.

Alternatively, the first display area may be arranged on the right side of the processing condition setting screen, and the second display area may be arranged on the left side of the processing condition setting screen.

In the first display mode, it is often required to set detailed sorting conditions, and accordingly, it is necessary to confirm detailed measurement data, or it is necessary to display a large number of pieces of plot data on one screen. On the other hand, it is also required to present the work progress status or the operation to be performed next to the first user. Therefore, as described above, by arranging the two areas on the left and right, a large second display area can be secured, whereby the first user can easily confirm the measurement data, and the first user can confirm the work situation by the display of the operation flow.

In order to improve the operability, in a case where the first display area is arranged on the left side of the processing condition setting screen and the second display area is arranged on the right side of the processing condition setting screen, the first display area may be arranged so as to be in contact with the left end of the processing condition setting screen, and the second display area may be arranged so as to be in contact with the right end of the processing condition setting screen.

Conversely, in a case where the first display area is arranged on the right side of the processing condition setting screen and the second display area is arranged on the left side of the processing condition setting screen, the first display area may be arranged so as to be in contact with the right end of the processing condition setting screen, and the second display area may be arranged so as to be in contact with the left end of the processing condition setting screen.

The arrangement of the first display area and the second display area may be changeable. For example, the biological particle sorting device may be designed to change the screen from a state where the former area is arranged on the right side and the latter area is arranged on the left side to a state where the former area is arranged on the left side and the latter area is arranged on the right side. Furthermore, the screen may be designed to be changeable so as to be opposite thereto. Therefore, it is possible to correspond to the dominant hand or convenience of the user, and it is possible to improve the operability.

### (First Display Area)

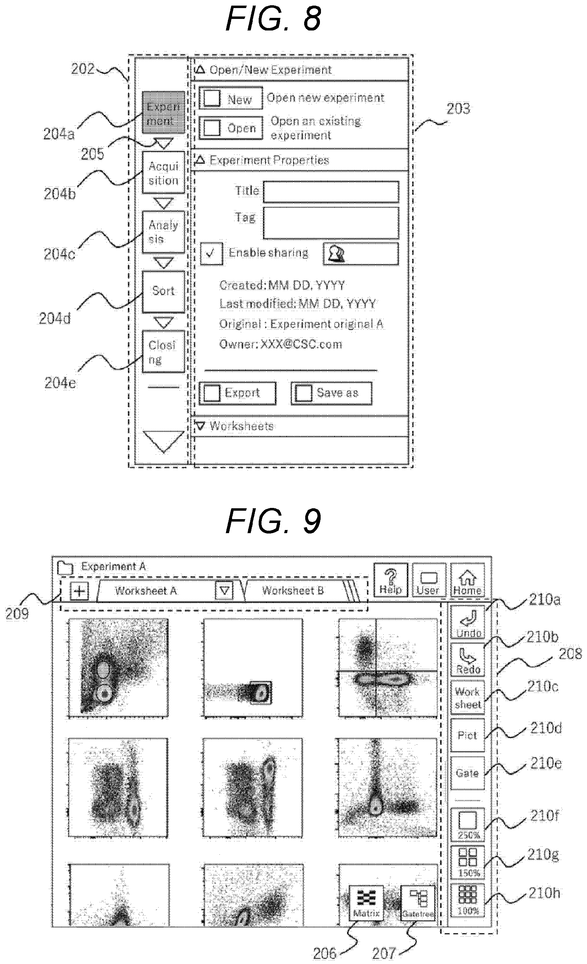

Fig. 8 shows an example of the first display area. As shown in the drawing, the first display area includes a procedure button area 202 and a setting information display area 203.

In the procedure button area, a plurality of processing buttons included in the operation flow is arranged in the order of processing.

In the drawing, the first display area is arranged at a left side end portion of the processing condition setting screen, and the procedure button area is arranged at a left side end portion in the first display area. Furthermore, the procedure button area is arranged on the left side of the first display area, and the setting information display area is arranged on the right side of the first display area.

Alternatively, the first display area may be arranged at the right side end portion of the processing condition setting screen, and the procedure button area may be arranged at the right side end portion in the first display area. Furthermore, the procedure button area may be arranged on the right side of the first display area, and the setting information display area may be arranged on the left side of the first display area.

Therefore, the first user can easily find each button in the workflow. In addition, since the procedure button area is arranged at the end (particularly, the left end) of the processing condition setting screen, when the processing button is touched, the possibility of touching other buttons is reduced, and an erroneous operation caused by touching an unintended portion can be reduced.

A user interface of a conventional biological particle sorting device is often assumed to be operated via a display of a desktop or laptop personal computer. In this case, for example, the cursor is operated by a mouse or the like, and the tool button which is frequently selected is arranged at the upper portion of the screen as in a window of general software.

However, as shown in Fig. 1 described above, in a case where the user operates the touch panel, if tool buttons which are frequently selected are arranged at the upper portion of the screen, it is difficult to touch the tool buttons. Furthermore, in a case where the finger is moved toward the tool buttons, the possibility of touching other buttons is also increased.

According to the present disclosure, the procedure button area is arranged on the left side (or the right side) of the screen, in particular, is arranged at the left end (or the right end), so that it is easy to access the buttons in the procedure button area, and there is a low possibility that other buttons are touched. Therefore, it is possible to provide a screen that is easy for the first user to use and operate.

The drawing is an example of the first display area in a case where the first display area is arranged on the left side of the processing condition setting screen and the second display area is arranged on the right side of the processing condition setting screen.

Note that, in a case where the first display area is arranged on the right side of the processing condition setting screen and the second display area is arranged on the left side of the processing condition setting screen, the first display area may be arranged so as to be in contact with the right end of the processing condition setting screen, and the procedure button area may be arranged on the right side of the first display area, and in particular, may be arranged on the right end of the processing condition setting screen.

The procedure button area 202 includes a plurality of processing buttons 204a to 204e, and each button indicates each of a plurality of processes performed from the start to the completion of the creation of the operation control data. As shown in the drawing, the plurality of processing buttons may be arranged from the top to the bottom of the screen. By arranging the plurality of processing buttons in one direction as described above, the order of each step can be grasped, and the progress status of the work can be easily grasped. Between the two processing buttons, a mark 205 (for example, a downward triangle, an arrow, or the like shown in the drawing) may be arranged to indicate that there is an order in these steps.

Among the plurality of processing buttons, a color or a shape of a button indicating a process in progress may be different from a color or a shape of another button. For example, as shown in the drawing, the processing button 204a indicating a process in progress may be shown in gray, and the processing buttons 204b to 204e indicating other processes may be shown in white. Therefore, it easy to specify the process in progress.

The shape of the plurality of processing buttons may be a polygon (for example, a rectangle) or a circle. The sizes and arrangement intervals of the plurality of buttons may be set such that two or more buttons are not selected in a case of touching with a fingertip, for example. In a case where each button is rectangular, the long side (or one side) may be, for example, 0.3 cm or longer, preferably 0.5 cm or longer. Furthermore, the long side (or one side) may be, for example, 5 cm or shorter, and preferably 3 cm or shorter. In a case where each button is circular, the diameter (or the major axis) may be, for example, 0.3 cm or longer, preferably 0.5 cm or longer. Furthermore, the diameter (or the major axis) may be, for example, 5 cm or shorter, and preferably 3 cm or shorter.

In the setting information display area 203, contents corresponding to the process selected by each procedure button are displayed. More specifically, the setting information display area 203 displays the setting information corresponding to any processing button in accordance with the touch of the processing button. An example of the displayed content will be described later.

### (Second Display Area)

In the second display area 251, measurement data is displayed as shown in Fig. 9. The second display area may be designed to display one or more plots, and may include a plurality of pieces of plot data, for example, as shown in the drawing. The plurality of pieces of plot data may be arranged in a grid shape. Furthermore, the display format of the plurality of pieces of plot data may be changed to a list form (for example, arranged from the top to the bottom). A gate can be set for each plot data. That is, the second display area may be designed to receive an input of gate information specifying the biological particle sorted in the processing by a touch operation.

In the second display area, one or more operation buttons may be displayed, and one or more operation buttons for displaying data associated with the one or more plots may be displayed.

The associated data is, for example, fluorescence correction matrix data. As an example of the operation button for displaying the fluorescence correction matrix data, a matrix display button 206 is shown in the drawing. The matrix display button (Matrix button) is a button for displaying a window for displaying and/or adjusting a fluorescence correction matrix used for generating plot data to be displayed in the measurement data area.

Furthermore, the associated data may be, for example, back data used to generate the plot. The back data may include, for example, statistical information of each gate. As an example of the operation button for displaying the statistical information, a gate tree display button 207 is shown in the drawing. The gate tree display button (Gatetree button) is a button for displaying a window for displaying a gate tree of plot data displayed in the measurement data area.

As shown in the drawing, these operation buttons may be arranged in the measurement data display area, and in particular, may be arranged in a right side portion (particularly, a lower right portion) of the second display area. Therefore, the user's finger can easily access.

Note that in a case where the second display area is arranged on the left side of the screen, these operation buttons may be arranged on the left side portion (particularly, the lower left portion) of the second display area. Furthermore, these buttons may be arranged in an operation button area described below.

Details of the window displayed by touching these operation buttons will be described later.

The second display area may further include an operation button area 208. Furthermore, the second display area may further include a tab area 209.

The operation button area is arranged on the right side of the second display area, and in particular, is arranged at the right end of the second display area. Therefore, the first user can easily find each operation button.

Furthermore, since the operation button area is arranged at the end (the right end) of the processing condition setting screen 200, when the operation button is touched, the possibility of touching another button is reduced, and an erroneous operation caused by touching an unintended portion can be reduced.

As described above, in a case where the user operates the touch panel, if tool buttons which are frequently selected are arranged at the upper portion of the screen, it is difficult to touch the tool buttons. Furthermore, in a case where the finger is moved toward the tool buttons, the possibility of touching other buttons is also increased.

According to the present disclosure, the operation button area is arranged on the right side (or the left side) of the screen, in particular, is arranged at the right end (or the left end), so that it is easy to access the buttons in the operation button area, and there is a low possibility that other buttons are touched. Therefore, it is possible to provide a screen that is easy for the first user to use and operate.

The drawing is an example of the second display area in a case where the first display area is arranged on the left side of the processing condition setting screen and the second display area is arranged on the right side of the processing condition setting screen.

Note that, in a case where the first display area is arranged on the right side of the processing condition setting screen and the second display area is arranged on the left side of the processing condition setting screen, the second display area may be arranged so as to be in contact with the left end of the processing condition setting screen, and the operation button area may be arranged on the left side of the set second display area, and in particular, may be arranged on the left end of the processing condition setting screen.

In the operation button area 208, a plurality of operation buttons 210a to 210h is displayed. Each operation button is a button for operating the measurement data displayed on the screen. As shown in the drawing, the plurality of operation buttons may be arranged in a line from the top to the bottom of the screen, but may be arranged in two or more lines, for example.

Note that, in the drawing, the operation buttons 210a to 210h are arranged in the operation button area, but one or more of these operation buttons may be arranged in the measurement data display area.

The shape of the plurality of operation buttons may be a polygon (for example, a rectangle) or a circle. The sizes and arrangement intervals of the plurality of buttons may be set such that two or more buttons are not selected in a case of touching with a fingertip, for example. In a case where each button is rectangular (rectangle shape or square), the long side (or one side) may be, for example, 0.3 cm or longer, preferably 0.5 cm or longer. Furthermore, the long side (or one side) may be, for example, 5 cm or shorter, and preferably 3 cm or shorter. In a case where each button is circular, the diameter (or the major axis) may be, for example, 0.3 cm or longer, preferably 0.5 cm or longer. Furthermore, the diameter (or the major axis) may be, for example, 5 cm or shorter, and preferably 3 cm or shorter.

The plurality of operation buttons may include, for example, an undo button 210a and a redo button 210b. The former is a button for canceling an operation on data on the screen. The latter is a button for redoing the operation on the data on the screen or executing the canceled operation again.

The plurality of operation buttons may include one or more toolbox display buttons. Examples of the toolbox display button include a worksheet toolbox display button 210c (Worksheet button), a plot toolbox display button 210d (Plot button), and a gate toolbox display button 210e (Gate button). The plurality of operation buttons may include one, two, or all three of them.

The worksheet toolbox button is a button for displaying a toolbox including a plurality of buttons for controlling a display format of the plot data displayed in the measurement data area.

The plot toolbox display button is a button for displaying a toolbox including a plurality of buttons for adjusting the content of each piece of plot data.

The gate toolbox display button is a button for displaying a toolbox including a plurality of buttons for adjusting gate information set to each piece of plot data.

Details of these toolboxes will be described later.

The plurality of operation buttons may further include a plurality of buttons for changing the display magnification of the plot data displayed in the second display area. For example, as shown in Fig. 9, the plurality of operation buttons may include a plurality of buttons 210f to 210h for displaying each piece of plot data at a predetermined magnification. Furthermore, the plurality of operation buttons may include a button for increasing the magnification and/or a button for decreasing the magnification.

The tab area 209 is an area in which one or more tabs for displaying only one of the plurality of open worksheets in the second display area are displayed. By selecting a certain tab, the tab is activated and the worksheets associated with the tab are displayed in the second display area. The worksheets associated with the inactive tabs are not displayed. In Fig. 9, a tab of the worksheet A is active, and a plot data group included in the worksheet A is displayed. In the drawing, the same experiment file also includes the worksheet B, but the worksheet B is inactive, and the plot data group included in the worksheet B is not displayed.

Herein, the worksheets associated with the active tab (also referred to as active worksheets) may mean the worksheets that are subject to the sorting processing. The biological particle sorting device may execute biological particle sorting processing according to gate information in the active worksheet.

Furthermore, inactive tabs are tabs associated with other worksheets other than the active worksheets. The worksheets associated with inactive tabs are also referred to as inactive worksheets. The inactive worksheets may be, for example, worksheets of sorting processing performed in the past and/or reference worksheets. The inactive tabs may be grouped and displayed at the ends (for example, right or left ends) of the tab area 209.

Preferably, the display position of the active tab in the tab area is fixed. For example, the tab area may be displayed such that the tab selected by the first user exists at the left end (or the right end) in the tab area. In this case, the inactive tab group is arranged at the right end (or the left end).

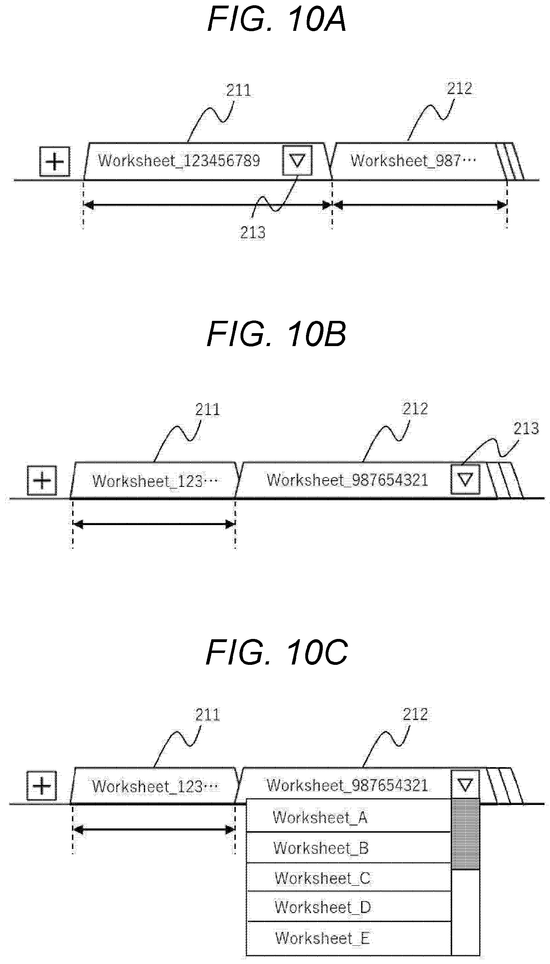

The size of the tabs may be constant, but is preferably dynamically changed. For example, the information processing unit 102 displays the size of the selected tab in the horizontal direction (arrangement direction of characters) to be larger than the size of the unselected tab in the horizontal direction. Therefore, the selected tab can be emphasized and presented to the user. For example, as shown in Fig. 10A, the size of the active tab 211 in the horizontal direction is displayed to be larger than the size of the inactive tab 212.

The size of the tab may be changed as the position of the tab is changed, or may be changed independent of the position of the tab, depending on whether or not the tab is selected.

Furthermore, the information processing unit 102 may change the size of the tab so that the full name of the worksheet of the active tab is displayed in the tab. Therefore, the selected tab can be emphasized and presented to the user. For example, in Fig. 10A, regarding the active tab 211, the full file name "Worksheet_123456789" is displayed. On the other hand, in Fig. 10B, the tab 211 is inactive, and accordingly, a part of the file name "Worksheet _123" and an ellipsis "..." are displayed in the tab 211.

The information processing unit 102 can display, in the tab area, for example, one active tab and one or two other tabs together with at least a part of the worksheet name. One or more other tabs may be designed to be displayed by pull-down, and may be designed to be displayed, for example, in a pull-down menu displayed by touching or selecting a predetermined button. Therefore, it possible to intuitively understand which is the active worksheet (particularly, the one being measured). Furthermore, even in a case where a large number of worksheets are opened in a narrow screen, it is possible to easily confirm a name of a worksheet to be compared or referred to, and it is possible to switch the worksheets by one touch or click.

For example, a pull-down button indicated by reference numeral 213 in Figs. 10A and 10B may be displayed in the tab area. By selecting the pull-down button, a pull-down menu may be displayed, for example, as shown in Fig. 10C. In the pull-down menu, worksheets that are not displayed in the tab area may be listed. Note that the pull-down button is arranged in the active tab in Figs. 10A and 10B, but may be arranged in the inactive tab or may be arranged at another position in the tab area. As described above, the information processing unit 102 may display, in the tab area, a pull-down button for displaying a list of worksheets that are not displayed by pull-down.

### (2-2-2) Example of Processing in First Display Mode

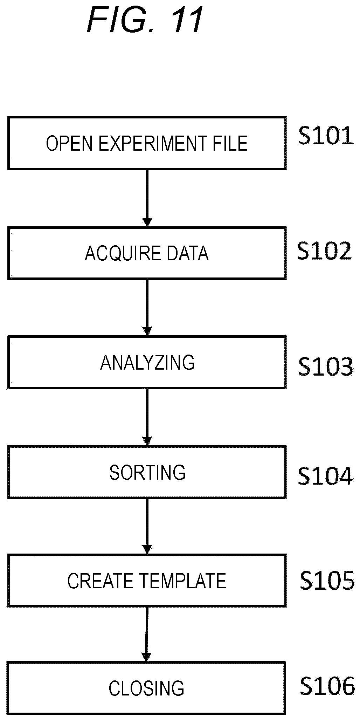

Fig. 11 shows an example of a flowchart in a case where the processing in the first display mode is performed in step S13 in (2-1) described above. An example of processing executed by the biological particle sorting device in the first display mode, in particular, a screen displayed in the processing will be described with reference to the drawing.

### (File Opening Process S101)

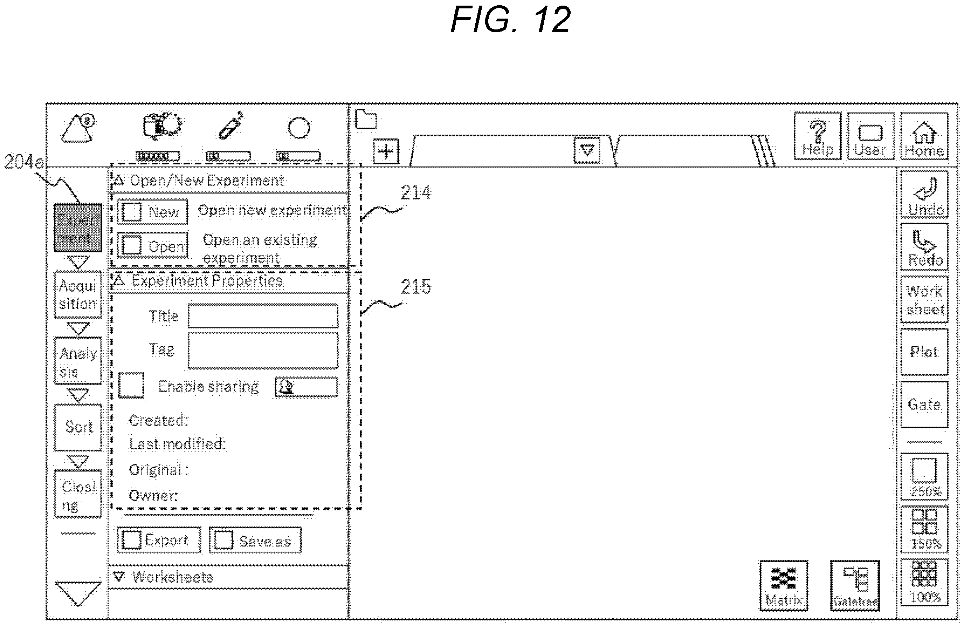

When the processing in step S13 is started, the information processing unit 102 causes the display unit 101 to display the processing condition setting screen shown in Fig. 12. At the start of the processing, as shown in the drawing, a processing button (shown as an Experiment button in the drawing) 204a for newly creating an experiment file or opening an existing experiment file may be selected in the first display area (particularly, the procedure button area).

In a state where the Experiment button is selected, as shown in the drawing, the information processing unit 102 displays, in the setting information display area, a file open area 214 (Open/New Experiment area) in which a button for newly creating an experiment file and a button for opening an existing experiment file are displayed, and a property area 215 (Experiment properties area) in which properties of the experiment file are displayed.

In accordance with the first user selecting a button for creating a new experiment file (shown as a New button in the drawing) in the file open area, the information processing unit 102 empties a title box (a box denoted as Title) and a tag box (a box denoted as Tag) in the property area, and empties the second display area. The title box is a field in which name data of the experiment file is input. The tag box is a field to which a tag (keyword) for searching an experiment file is input.

The first user inputs title data and tag data in the property area. The input title data and tag data may be included in the operation control data.

In accordance with selection of a button for opening an existing experiment file (indicated as an Open button in the drawing) by the first user, the information processing unit 102 displays a screen for permitting the first user to select the existing experiment file. In accordance with the first user selecting the existing experiment file on the screen, the information processing unit 102 outputs the title data and the tag data included in the existing experiment file to the title box and the tag box, respectively.

For example, on the processing condition setting screen shown in Fig. 6, "Experiment A" is output as the experiment file name, and "foo, bar, baz" is output as the tag.

As shown in the drawing, other property data related to the operation control data may be displayed in the property area. The other property data may include, for example, a creation date and a last update date of the operation control data. The other property data may also be included in the operation control data.

As shown in the drawing, the setting information display area may further display an Export button (shown as an export button in the drawing) for exporting the opened operation control data and/or a save button (shown as a Save button in the drawing) for saving the operation control data.

For example, after inputting property data in the property area in a case where a new experiment file is created or confirming property data in the property area in a case where an existing experiment file is opened, the first user selects the Acquisition button in the procedure button area in order to acquire data on the biological particle-containing sample.

### (Data Acquisition Process S102)

In accordance with the selection of the Acquisition button, the information processing unit 102 changes the color of the button in the first display area (particularly, the procedure button area) so as to indicate a state where the Acquisition button is selected.

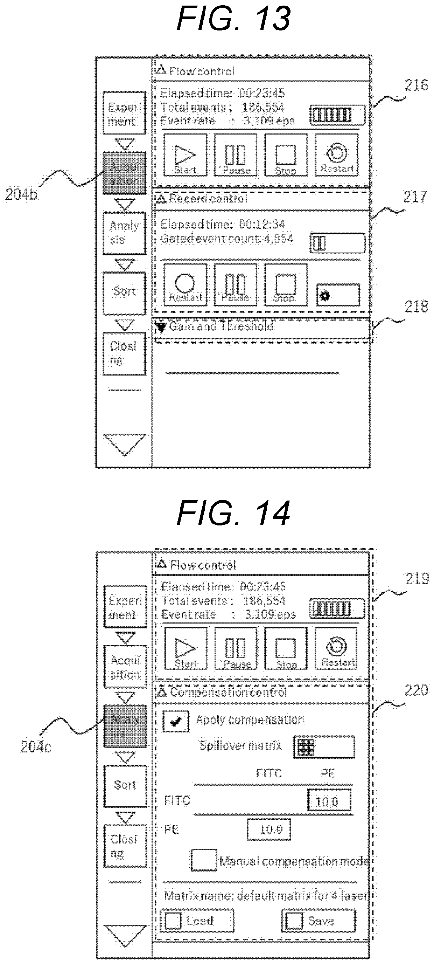

In accordance with the selection of the Acquisition button, the information processing unit 102 displays a screen as shown in Fig. 13 in the setting information display area. The screen is a screen for controlling processing of acquiring data (light data, in particular, scattered light data and/or fluorescence data) on the biological particles in the biological particle-containing sample. In the above state, the information processing unit 102 displays a flow control area 216 (Flow control area) and a recording control area 217 (Record control area) in the setting information display area as shown in the drawing.

In the flow control area, as shown in the drawing, a group of buttons for controlling the operation of flowing the biological particle-containing sample through the flow channel in which the sorting processing is performed, and data indicating the status of the operation are displayed. As shown in the drawing, the button group includes a start button (Start button) for starting the operation of flowing the sample (or restarting the paused operation), a pause button (Pause button) for pausing the operation, an end button (Stop button) for terminating the operation, and a restart button (Restart button) for restarting the terminated operation. Furthermore, the elapsed time of the operation, the number of events detected in the operation, the detection rate (the number of times of event detection per unit time), and the like may be displayed in the flow control area.

In the recording control area, as shown in the drawing, a button group for controlling recording of a sorting determination result based on a set gate and data indicating a status of the recording are displayed. As shown in the drawing, the button group includes a start button (Record button) for starting the recording (or starting again recording that has been paused), a pause button (Pause button) for pausing the recording, and an end button (Stop button) for ending the recording. Furthermore, in the recording control area, the elapsed time of the recording operation, the number of events to be analyzed, and the like may be displayed.

Furthermore, in a state where the Acquisition button is selected, the biological particle sorting device 100 may display an area 218 for setting a gain and/or a threshold in the setting information display area. The biological particle sorting device 100 may be designed to be able to set a gain and/or a threshold in the detection unit in the area. With these settings, it is possible to control the type or range of the event to be analyzed. Note that, in the drawing, the area is closed. An area for performing the setting is expanded in accordance with the user selecting the area.

The first user inputs settings related to the gain and/or the threshold in this area. The input setting data related to the gain and/or the threshold may be included as a part of the operation control data, and in particular, may be handled as a part of the "(a) processing condition data adopted by the processing device that executes the processing in the second mode".

The first user selects the Analysis button 204c in the procedure button area after the light detection data of a predetermined number of events is acquired. Alternatively, when data of a preset number of events is obtained, the information processing unit 102 may automatically shift to a state where the Analysis button 204c is selected.

### (Analysis Process S103)

In accordance with the selection of the Analysis button, the information processing unit 102 changes the color of the button in the first display area (particularly, the procedure button area) so as to indicate a state where the Analysis button is selected.

In accordance with the selection of the Analysis button, the information processing unit 102 displays a screen as shown in Fig. 14 in the setting information display area. The screen is a screen for setting a gate for sorting target biological particles on the basis of the optical data acquired in the data acquisition process. In a state where the Analysis button is selected, the biological particle sorting device 100 displays a flow control area 219 and a fluorescence correction setting area 220 (displayed as a Compensation control area in the drawing) in the setting information display area.

The flow control area 219 is the same as the flow control area 216 described above with respect to the Acquisition step, and a group of buttons for controlling the operation of flowing the biological particle-containing sample through the flow channel in which the sorting processing is performed and data indicating the status of the operation are displayed.

In the fluorescence correction setting area 220, as shown in the drawing, a check box (displayed as Apply compensation in the drawing) for selecting whether to apply fluorescence correction and one or more correction coefficient input boxes (in the drawing, a numerical value 10.0 is input) for adjusting a correction coefficient included in the read correction matrix are displayed. The correction matrix is adjusted by inputting a numerical value into the correction alphanumeric input box.

Furthermore, in the area, a name of a correction matrix to be applied (displayed as default matrix for 4 laser in the drawing), a button (Load button) for reading the correction matrix, and a button (Save button) for saving the adjusted correction matrix in a case where the read correction matrix is adjusted may be displayed.

In this area, the first user inputs settings (correction coefficients and the like) related to the correction matrix. The input setting data related to the correction matrix may be included in the operation control data, and in particular, may be included as a part of the "(a) processing condition data adopted by the processing device that executes the processing in the second mode".

In a state where the Analysis button is selected, the first user performs gate setting in the second display area and inputs gate information. The input gate information may be included in the operation control data, and in particular, may be included as a part of the "(a) processing condition data adopted by the processing device that executes the processing in the second mode".

The setting of the gate information may be appropriately executed by the first user. For example, the plot data is generated from the acquired optical data, the gate is set for the generated plot data, the gate is developed to further generate plot data, and then the gate is further set for the plot data. By appropriately repeating the operations of the plot data generation, the gate setting, and the development in this manner, the gate information for sorting a target biological particle is generated. The generated plot data may be a two-dimensional plot or a one-dimensional plot, and the type of the plot data may be appropriately selected by the first user. Furthermore, the scattered light/fluorescence channel and the scale of the axis adopted as the axis of each plot data may be appropriately selected by the first user.

The information processing unit 102 displays one or more pieces of plot data generated for setting the gate information described above in the second display area. The generated one or more pieces of plot data may be included in the operation control data. For example, any one or more pieces of image data of the generated one or more pieces of plot data may be included in the operation control data, in particular, it may be included as a part of "operator-oriented instruction data directed to an operator operating the processing device in the second mode" in above (c), and more particularly, may be included as data to be referred to by the second user.

In the generation of the gate information, a window displayed by selecting the matrix display button and/or the gate tree display button may be referred to. These will be described below.

### (Matrix Display Button)

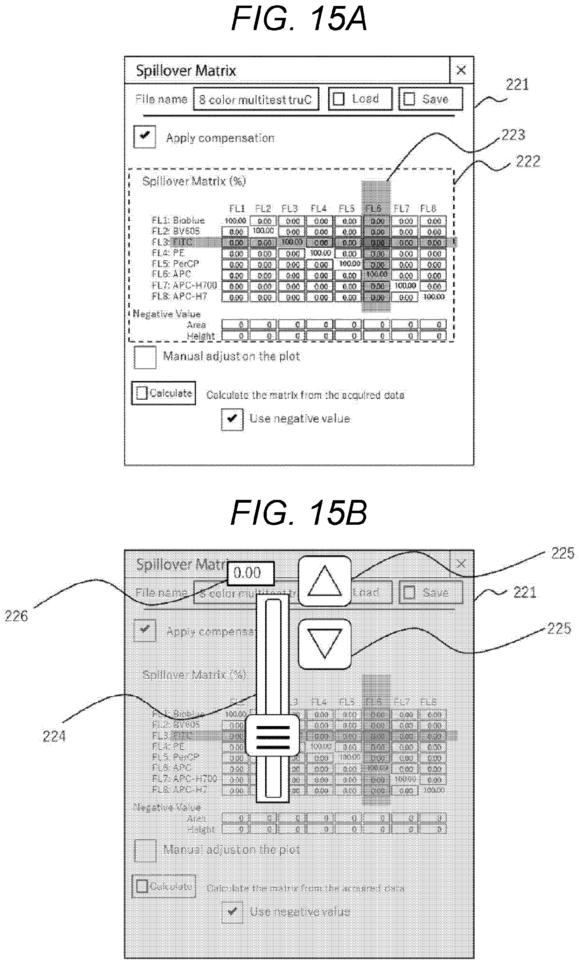

A window displayed by selecting the matrix display button is shown in Fig. 15A. As shown in the drawing, the window 221 includes a correction matrix 222 applied to the displayed measurement data. The size of the window may be smaller than the size of the second display area. For example, the area of the window may be, for example, 60% or less, preferably 50% or less of the area of the second display area. Furthermore, the area of the window may be, for example, 20% or more, preferably 30% or more of the area of the second display area. Therefore, the parameters in the correction matrix can be adjusted while checking the measurement data displayed in the second display area.

Furthermore, as shown in the drawing, the window may include a file name display field (denoted as File name in the drawing) for displaying a file name of the correction matrix, a read button (Load button) for reading the correction matrix, and a save button (Save button) for saving the correction matrix after adjustment.

As shown in the drawing, the window may further include a check box for selecting whether to apply correction, a check box for selecting whether to perform manual adjustment on the plot, a Calculate button for calculating a matrix from acquired data, and a check box for selecting whether to utilize Negative Value.

In accordance with the first user selecting one of the correction coefficients in the correction matrix, the information processing device 102 may perform image processing of emphasizing the selected correction coefficient, such as displaying a cross-shaped indicator 223 for emphasizing the selected correction coefficient. Therefore, the first user can easily confirm the correction coefficient selected by the first user.

The information processing unit 102 may be designed to receive adjustment of each correction coefficient in the correction matrix. A situation of the adjustment is shown in Fig. 15B. As shown in the drawing, when the finger of the first user selects the correction coefficient to be adjusted, the information processing device 102 displays a slider 224 in the vicinity of the selected position. Accordingly, the information processing device 102 may execute image processing of emphasizing the slider, such as adding gray color to the window 221 as shown in the drawing.

As shown in the drawing, the slider may be an indicator movable in the vertical direction, or may be an indicator movable in the horizontal direction or any other two directions.

The information processing unit 102 may display a slider movement button 225 for moving the slider in the vicinity of the slider. The slider may be moved in accordance with selection of the button.

The information processing unit 102 may enlarge and display the box 226 displaying the selected correction coefficient in the vicinity of the slider. Therefore, the first user can easily confirm the value of the correction coefficient value selected by the first user. The information processing device 102 changes the value of the correction coefficient in the box in accordance with the slider movement or the touch of the button.

The first user may adjust the correction matrix by operating the window. In accordance with the first user selecting the save button after the adjustment, the information processing device 102 saves the adjusted correction matrix data. The adjusted correction matrix data may be included in the operation control data, and in particular, may be included as a part of the "(a) processing condition data adopted by the processing device that executes the processing in the second mode".

Preferably, the display or non-display of the window may be switched by touching a matrix display button, that is, the matrix display button may be a toggle button. In accordance with the matrix display button being touched by the user, the information processing unit 102 displays the window in the processing condition setting screen, and thereafter, in response to the matrix display button being further touched by the user, the information processing unit 102 closes the displayed window.

While it is assumed that the window is frequently confirmed by the first user, it is difficult to confirm the plot data in the measurement data display area if the window is always displayed. Therefore, by using the matrix display button as the toggle button as described above, it is possible to improve convenience of the window while facilitating confirmation of the plot data.

The matrix display button is preferably arranged at an end of the processing condition setting screen, in particular, at an end of the second display area. Therefore, this makes it easier for the user to touch the matrix display button. In Fig. 9, the matrix display button 206 is arranged in the measurement data display area in the second display area, but the button may be arranged in the operation button area.

### (Gate Tree Display Button)

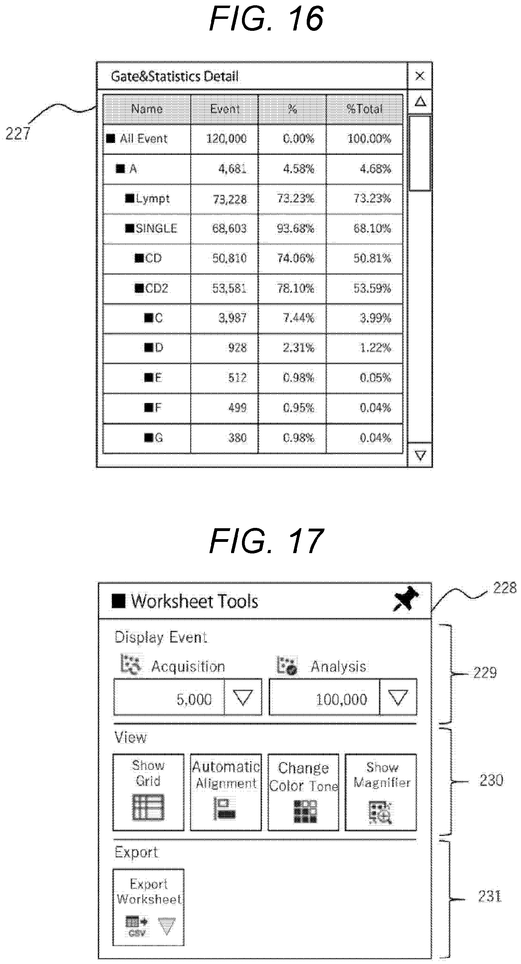

An example of a window displayed by selecting the gate tree display button is shown in Fig. 16. A window 227 shown in the drawing indicates the statistical data of the displayed measurement data group. The size of the window may be smaller than the size of the second display area. For example, the area of the window may be, for example, 60% or less, preferably 50% or less of the area of the second display area. Furthermore, the area of the window may be, for example, 20% or more, preferably 30% or more of the area of the second display area. Therefore, the parameters in the correction matrix can be adjusted while checking the measurement data displayed in the second display area.

The statistical data includes the name of each gate. Moreover, the statistical data may include one, two, or all three of the number of events included in each gate, the ratio of the number of events included in each gate among the number of events displayed in certain plot data, and the ratio of the number of events included in each gate with respect to the total number of events. In the statistical data shown in the drawing, the name of each gate includes the number of events corresponding to each gate and two types of the ratio described above.

The information processing unit 102 may be designed to change the statistical data in accordance with adjustment of each gate. Since the statistical data is frequently checked in the biological particle sorting processing, the first user can efficiently check the influence of the gate adjustment by changing the statistical data in conjunction with the gate adjustment.

Preferably, the display or non-display of the window may be switched by touching a gate tree display button, that is, the gate tree display button may be a toggle button. In accordance with the gate tree display button being touched by the user, the information processing unit 102 displays the window in the processing condition setting screen, and thereafter, in response to the gate tree display button being further touched by the user, the information processing unit 102 closes the displayed window.

While it is assumed that the window is also frequently confirmed by the first user, it is difficult to confirm the plot data in the measurement data display area if the window is always displayed. Therefore, by using the gate tree display button as the toggle button as described above, it is possible to improve convenience of the window while facilitating confirmation of the plot data.

The gate tree display button is preferably arranged at an end of the processing condition setting screen, in particular, at an end of the second display area. Therefore, this makes it easier for the user to touch the gate tree display button. In Fig. 9, the gate tree display button 207 is arranged in the measurement data display area in the second display area, but the button may be arranged in the operation button area.

In order to generate the gate information, a toolbox group that is displayed by selecting the worksheet toolbox display button, the plot toolbox display button, and the gate toolbox display button may be utilized. These will be described in below.

### (Worksheet Toolbox)

An example of a toolbox displayed by selecting the worksheet toolbox display button is shown in Fig. 17. The toolbox 228 shown in the drawing includes a button group for adjusting settings applied to the entire one worksheet or performing processing on the entire one worksheet.

For example, as shown in the drawing, the toolbox may include one, two, or three of an area 229 including one or more buttons for adjusting the number of events to be displayed on the worksheets, an area 230 including one or more buttons for adjusting the display format of the plot data in the worksheets, and an area 231 including one or more buttons for exporting the worksheets.

The first user uses the toolbox to set the worksheets. As a result, the worksheet setting data is generated. That is, the worksheet setting data includes any one or more pieces of data input via the toolbox described above. The data input in this manner may be included as a part of the operation control data, for example, may be included as a part of the "(b) display control data related to display on the processing execution operation screen in the second mode". For example, number-of-events data (data input via the area 229) for specifying the number of events to be displayed, display format data (data input via the area 230) for specifying the display format of the plot data, or both of them may be included in the display control data of (b) above. Note that, these pieces of data may be handled as being included in the "(a) processing condition data adopted by the processing device that executes the processing in the second mode".

### (Plot Toolbox)

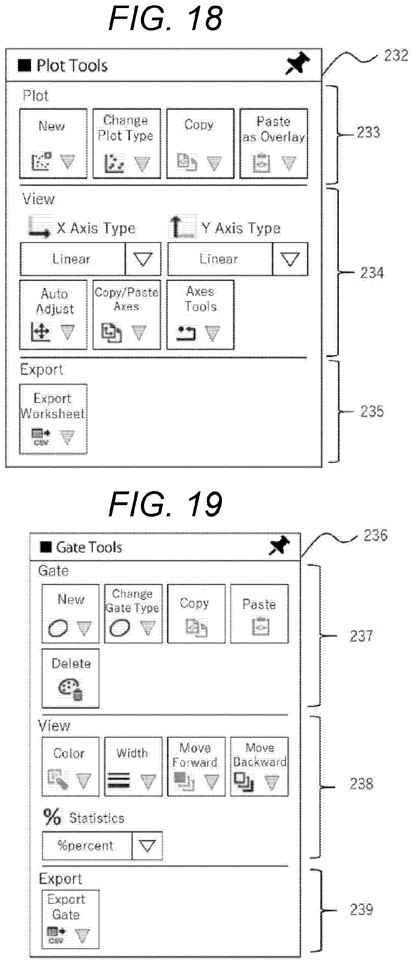

An example of a toolbox displayed by selecting the plot toolbox display button is shown in Fig. 18. The toolbox 232 shown in the drawing includes a button group for adjusting settings applied to each piece of plot data or operating each piece of plot data.

For example, as shown in the drawing, the toolbox may include one, two, or three of an area 233 including one or more buttons for operating plot data (for example, including a plot new creation button, a plot format change button, a plot data copy button, a plot data paste button (for overlay analysis), and the like), an area 234 including one or more buttons for adjusting axes of plot data (for example, a scale setting (log scale or linear scale) change button of each axis, an automatic adjustment button of both axes, an axis copy/paste button, an axis adjustment tool button, and the like), and an area 235 including one or more buttons for exporting a worksheet.

The first user uses the toolbox to set each plot. As a result, the plot setting data is generated. That is, the plot setting data includes any one or more pieces of data input via the toolbox described above. The data input in this manner may be included as a part of the operation control data, for example, may be included as a part of the "(b) display control data related to display on the processing execution operation screen in the second mode". For example, axis specifying data (data input via the area 234) specifying a display format of an axis of the plot data may be included in the display control data of (b) above. Note that, these pieces of data may be handled as being included in the "(a) processing condition data adopted by the processing device that executes the processing in the second mode".

### (Gate Toolbox)

An example of a toolbox displayed by selecting the gate toolbox display button is shown in Fig. 19. The toolbox 236 shown in the drawing includes a button group for setting a gate to each piece of plot data.

For example, as shown in the drawing, the toolbox may include one, two, or three of an area 237 including one or more buttons for operating each gate (for example, including a new gate creation button, a gate format change button, a gate data copy button, a gate pasting button, a gate deleting button, and the like), an area 238 including one or more buttons for changing the display format of each gate (for example, including a color change button of a line defining a gate, a width change button of the line, a button for changing an anteroposterior relationship of gate groups overlapping each other, a setting change button of statistical processing for an event included in each gate, and the like), and an area 239 including a button for exporting data (statistical data or the like) related to each gate.

The first user uses the toolbox to set each gate. As a result, the gate setting data is generated. That is, the gate setting data includes any one or more pieces of data input via the toolbox described above. The data input in this manner may be included as a part of the operation control data, for example, may be included as a part of the "(b) display control data related to display on the processing execution operation screen in the second mode". For example, the generated gate specifying data (data input via the area 237) specifying the gate, the generated data (data input via the area 238) specifying the display format of the gate, or both of them may be included in the display control data of (b) above. Note that, these pieces of data may be handled as being included in the "(a) processing condition data adopted by the processing device that executes the processing in the second mode".

### (Operation Panel Displayed near Plot)

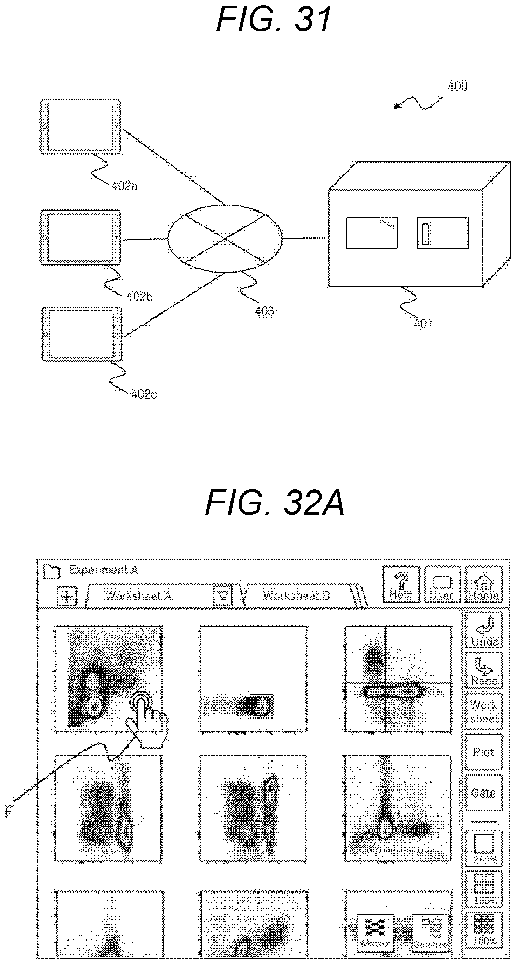

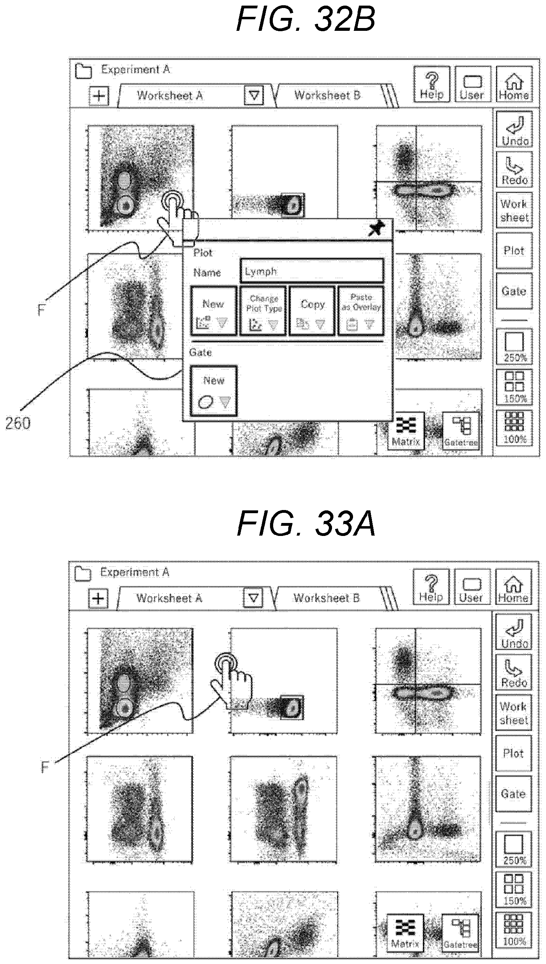

In a preferred embodiment of the present disclosure, in accordance with a touch on any plot in the second display area, the information processing unit 102 displays an operation panel for operating the plot in the vicinity of the touched position. An example of the operation panel is shown in Figs. 32A and 32B.

As shown in Fig. 32A, a plurality of plots is displayed in the second display area. It is assumed that a finger F of the first user touches one of the plots. In accordance with the plot being touched, the information processing unit 102 displays the operation panel 260 in the vicinity of the plot as shown in Fig. 32B. More specifically, the position where the operation panel is displayed may be near the touched position.

The information processing unit 102 may display the operation panel 260 within 5 cm, preferably within 4 cm, more preferably within 3 cm, and still more preferably within 2 cm from the touched position, for example. The distance may mean a distance between the touched position and a point closest to the touched position in the area of the operation panel.

The operation panel may include one or more operation tool selection buttons. The operation tool selection button may include, for example, one or more tool buttons frequently used by the user. Preferably, the operation tool selection button included in the operation panel may be designed to be changeable by the user. The operation tool selection button includes, for example, any one or more buttons included in the plot toolbox and/or any one or more buttons included in the gate toolbox. The operation panel 260 shown in Fig. 32B includes the plot new creation button, the plot format change button, a plot data copy button, and the plot data paste button among the buttons mentioned for the plot toolbox, and also includes the gate new creation button among the buttons mentioned for the gate toolbox.

Furthermore, different operation panels may be displayed in the vicinity of the touch position in accordance with the touched plot portion. For example, in accordance with a touch on the X-axis portion of the plot, the information processing unit 102 may display an operation panel (the toolbox) for performing X-axis adjustment in the vicinity of the touch position. Furthermore, in accordance with a touch on the Y-axis portion of the plot, the information processing unit 102 may also display an operation panel (the toolbox) for performing Y-axis adjustment in the vicinity of the touch position. Furthermore, in accordance with the touch of the inside of the frame of the gate, the information processing unit 102 may display an operation panel (the tool box) for gate adjustment near the touch position.

### (Axis Operation Slider Bar)

In a preferred embodiment, the information processing unit 102 displays a slider bar for changing the display format of any axis of the plot in the second display area in accordance with the axis being touched. The slider bar is a slider bar for adjusting a numerical range or scale of each axis. The axes (X-axis and Y-axis) of each piece of plot data may be axes in any format of Biexponential, Linear, or Log.

For example, three slider bars are displayed for Biexponential axis setting. An example of the slider bar displayed for setting a Biexponential axis will be described with reference to Figs. 33A to 33D.

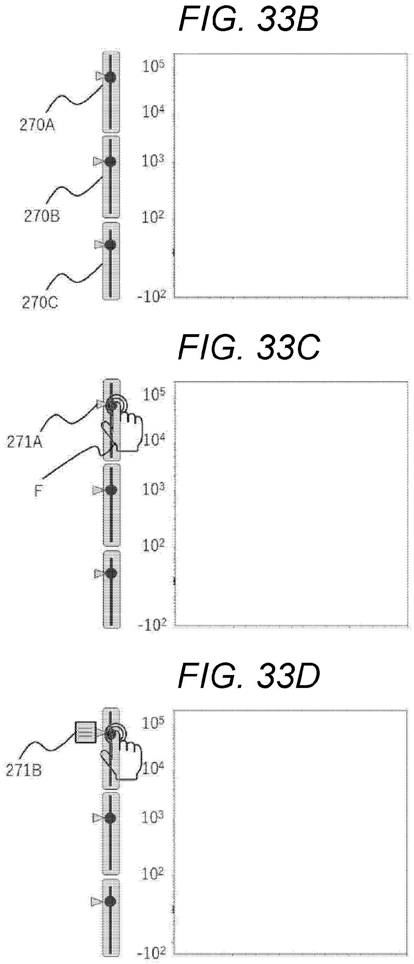

As shown in Fig. 33A, the first user touches any axis portion of the plot data. In accordance with the axis portion being touched by the finger F of the user, as shown in Fig. 33B, the information processing unit 102 displays three slider bars 270A, 270B, and 270C beside the axis of the plot data. Among them, the slider bar 270A is a slider bar used to adjust the maximum value of the axis, the slider 270B is a slider bar used to adjust the width near 0 of the axis, and the slider bar 270C is a slider bar used to adjust the minimum value of the axis. The information processing unit 102 changes the plot data (display of axes and event data) in accordance with a drag operation of a knob of each slider bar. Note that even in a case where the drag operation is moved upward or downward beyond the display range of the slider bar, the change of the plot data may be continued. Therefore, for example, even in a case where the plot data is displayed small, it is easy to adjust the axis. Although the above-described three types of slider bars are displayed in Fig. 33B, the information processing unit 102 may display any one or two of these three types.

Note that, in accordance with a region other than the slider bar being touched, the information processing unit 102 deletes the slider.

As shown in Fig. 33C, the first user touches one of the slider bars. In accordance with the slider bar being touched by the finger F of the user, the information processing unit 102 changes the display format of the knob 271 of the slider bar as shown in Fig. 33D. As shown in Figs. 33C and 33D, the change is preferably such that knob 271B after the touch is larger than knob 271A before the touch and/or such that the shape of knob 271B after the touch is different from the shape of knob 271A before the touch.

Furthermore, one slider bar may be displayed for setting the Linear axis or the Log axis. The information processing unit 102 may change the numerical range of the axis in accordance with the movement of the knob of the one slider bar.

By adopting the slider bar as described above, the operability of the shaft operation by touch can be improved.

### (Display of Enlarged Image)

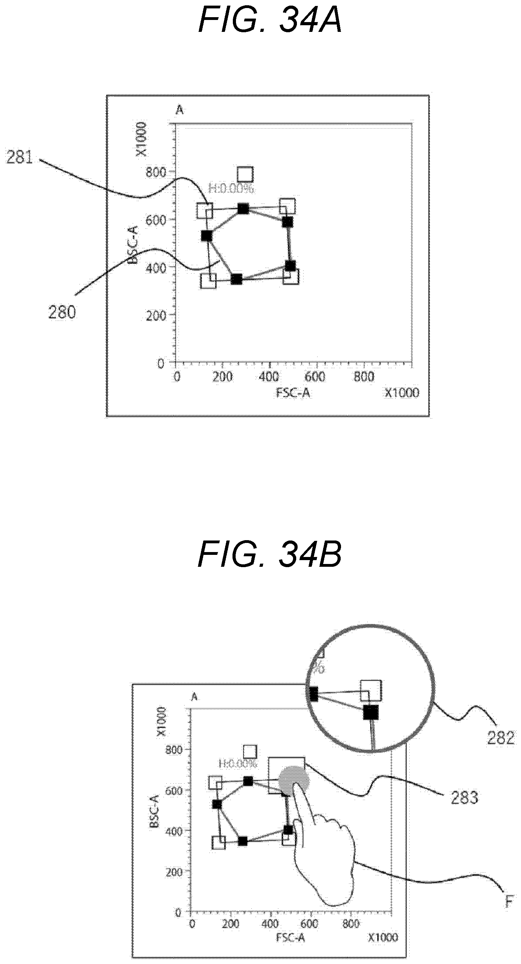

The information processing unit 102 can display an enlarged image of the touched portion in accordance with the touch of the data display portion of any of the plots in the second display area. Preferably, in accordance with any gate set in the plot data (particularly, a handle set in the gate) being touched by the user, the information processing unit 102 displays an enlarged image of the touched position in the vicinity of the position. The display of the enlarged image will be described below with reference to Figs. 34A and 34B.

A gate 280 is set in the plot data shown in Fig. 34A. Moreover, a plurality of handles 281 (indicated by squares) is also set in the gate 280 in advance. When one of the handles is touched by the finger F of the user, in accordance with the touch of the handle, as shown in Fig. 34B, the information processing unit 102 displays an enlarged image 282 centered on the position of the handle in the vicinity of the touched position or in the vicinity of the plot data. Furthermore, with the display of the enlarged image 282, the information processing unit 102 displays the touched handle in a large size as indicated by reference numeral 283.

Furthermore, the entire gate is selected in accordance with a touch on a portion (a line defining the gate or an inner side thereof) other than the handle in the gate 280, and the information processing unit 102 moves the entire gate by, for example, a drag operation.

The shape of each handle may be polygonal (particularly, rectangular) or circular, or other shapes. The size of the size (long side of rectangle shape or one side of square, diameter of circle) of each handle may be, for example, 5 dots to 25 dots, preferably 10 dots to 20 dots in the non-touched state, and may be, for example, 30 dots to 60 dots, preferably 40 dots to 55 dots in the touched state (the state displayed large as described above). Furthermore, the size of the handle in the touched state may be 1.5 to 5 times, preferably 2 to 4 times the size of the handle in the untouched state. By displaying the touched handle in a large size in this manner, the user can easily identify the touched handle.

The content to be displayed on the processing condition setting screen is diverse, and thus the size of the area where the plot data is displayed is also limited, and accordingly, each plot data is often displayed small. On the other hand, the gate set in the plot data is required to be precisely adjusted. By displaying the enlarged image as described above, fine gate adjustment can be performed even in a case where the plot data is displayed small.

### (Sorting Process S104)

For example, in accordance with the selection of the Sort button by the first user after the setting of the sorting gate information is completed in the Analysis step, the biological particle sorting device 100 changes the color of the button so as to indicate a state where the Sort button is selected in the first display area (particularly, the procedure button area).

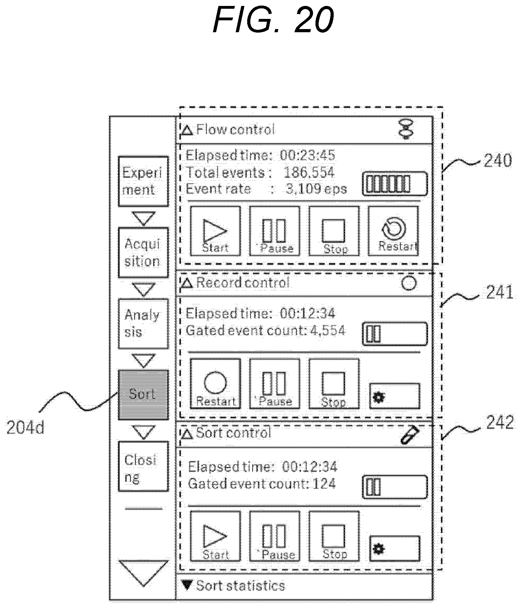

In accordance with the selection of the Sort button by the first user, the biological particle sorting device 100 displays a screen for controlling the sorting processing by the biological particle sorting device 100 in the setting information display area as shown in Fig. 20. In a state where the Sort button is selected, the biological particle sorting device 100 displays a flow control area 240, a recording control area 241, and a sorting control area 242 in the setting information display area.

The flow control area and the recording control area are the same as those described above with respect to the Acquisition step.

As shown in the drawing, the sorting control area includes a start button (Start button) for starting the sorting processing, a pause button (Pause button) for pausing the sorting processing, and an end button (Stop button) for ending the sorting processing. Furthermore, in the sorting control area, the elapsed time of the sorting operation (Elapsed time), the number of times the sorting is executed in the sorting operation (Gated event count), and the like may be displayed.

### (Template Creation Process S105)

In accordance with the user selecting the Template button 204g at an arbitrary timing, the biological particle sorting device 100 changes the color of the button so as to indicate a state where the Template button is selected in the first display area (particularly, the procedure button area). In Fig. 11, the template creating process S105 is shown to be executed, for example, after the processing in the sorting process S104, but the template creating process S105 may be executed after any other process (S101, S102, S103, or S105).

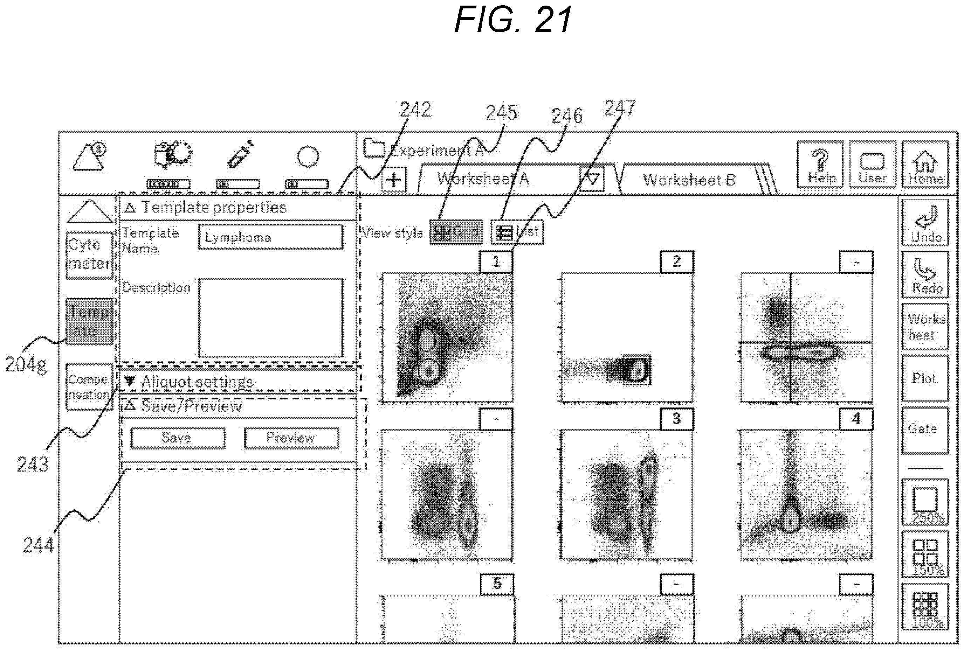

In accordance with the selection of the Template button by the user, the biological particle sorting device 100 displays the template property area 242 in the setting information display area as shown in Fig. 21. The template property area includes a template name input field (Template name field) and an input field for description of the template (Description field). Furthermore, in the template property area, for example, the number of times of revision and/or the creation date of the template may be displayed.

The first user inputs template property data (a template name and/or a description related to a template) in the template property area. As a result, template property data is generated. That is, the template property data includes any one or more pieces of data input via the template property area described above. The data input in this manner may be included in the operation control data, and in particular, may be included as a part of the "(c) operator-oriented instruction data directed to an operator operating the processing device in the second mode". For example, template specifying data including a template name and/or a description related to a template may be included in the operator-oriented instruction data of (c) above. Furthermore, the template specifying data may further include the number of times of revision and/or a creation date of the template.

Furthermore, as shown in the drawing, an aliquot setting button 243 is also displayed in the setting information display area. By selecting the button, an aliquot setting area to be described later is expanded in the setting information display area. The aliquot setting area may be developed in the setting information display area in advance.

In the present specification, an aliquot (Aliquot) means a sample (particularly, a biological particle-containing sample) which is sorted until temporarily stopping the sorting processing when temporarily stopping the sorting processing at a predetermined timing in the middle of the sorting processing. The aliquot is subjected to analysis by, for example, a device or an analysis kit different from the biological particle sorting device 100, or by a device or an analysis kit attached to the biological particle sorting device 100. As a result of the analysis, in a case where the aliquot satisfies a predetermined condition, the sorting processing is resumed. By acquiring and analyzing the aliquot, it is possible to confirm whether the biological particle obtained by the sorting processing satisfies a predetermined condition, and it is possible to determine whether to continue the sorting processing or adjust the sorting condition. Furthermore, it is also possible to avoid execution of useless sorting processing.

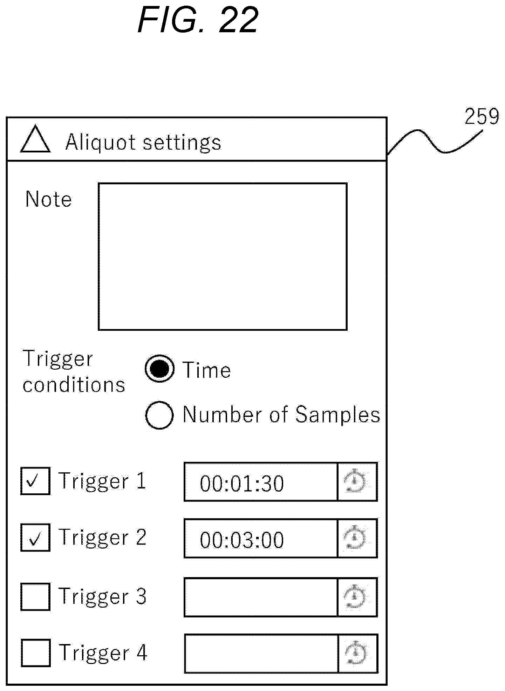

An example of a screen of an aliquot setting area is shown in Fig. 22. The aliquot setting area 259 shown in the drawing includes a field for inputting an instruction for an operator regarding acquisition of an aliquot and a timing setting field for setting one or more timings for acquiring an aliquot. The timing setting field includes a field for selecting whether to acquire an aliquot at a specific time point or to acquire an aliquot with a specific number of samples, and a field for specifying the time point or the number of samples.

The first user inputs the aliquot setting data (for example, aliquot acquisition instruction data including an instruction for an operator regarding aliquot acquisition and/or timing setting data for setting the timing) to the aliquot setting area. As a result, the aliquot setting data is generated. That is, the aliquot setting data includes any one or more pieces of data input via the aliquot setting area described above. The data input in this manner may be included as a part of the operation control data, and may be included, for example, as a part of the "(a) processing condition data adopted by the processing device that executes the processing in the second mode" or "(c) operator-oriented instruction data directed to an operator operating the processing device in the second mode". For example, the aliquot acquisition instruction data may be included in the operator-oriented instruction data of (c) above. Furthermore, the timing setting data may be included in the processing condition data of (a) above.

In a state where the Template button is selected, a grid display button 245 for arranging a plurality of pieces of measurement data (particularly, the plot data) in a grid pattern and a list display button 246 for arranging the plurality of pieces of measurement data in a list pattern are displayed in the second display area. Note that when the Template button is selected, either the grid display button or the list display button may be selected by default.

In accordance with the selecting the grid display button by the user, the biological particle sorting device 100 displays an order designation field 247 for designating the adjustment order in the second display mode in the vicinity of each measurement data as shown in Fig. 21. As shown in the drawing, the order designation field may be displayed so as to be in contact with each measurement data. The order of the plot data operated by the second user in the second display mode can be controlled by the order data input in the order designation field, and the second user can perform gate adjustment in an appropriate order.

The order designation field may be designed such that, for example, any positive integer can be selected or may be designed such that any positive integer can be input. The positive integer corresponds to the adjustment order. For example, in the drawing, "1" is selected for the left plot data and "2" is selected for the center plot data among the three pieces of plot data in the first row.

Furthermore, there is a case where the measurement data that does not need to be displayed in the second display mode may exist. In order to cope with this case, for example, the order designation field may be designed to permit selection of an option indicating that display is unnecessary, such as "-" or "display unnecessary". In Fig. 21, for example, "-" indicating that display is unnecessary is selected for the right plot data among the three pieces of plot data in the first row. Note that the order designation field may be designed to permit selection of an option indicating that display is necessary.

The first user inputs or selects an adjustment order or inputs or selects necessity of display (particularly, unnecessary of display) in an order designation field of each measurement data (the plot data) displayed in the second display area. Therefore, the measurement data display setting data is generated. That is, the measurement data display setting data includes the data input through the order designation field in this manner, and includes, for example, adjustment order data or display necessity data. The generated measurement data display setting data may be included as a part of the operation control data, and in particular, may be included as a part of the "(b) display control data related to display on the processing execution operation screen in the second mode". Note that the measurement data display setting data may be handled as being included in the "(c) operator-oriented instruction data directed to an operator operating the processing device in the second mode". Such measurement data display setting data is useful for accurately notifying the user in the second display mode of an instruction related to the sorting processing.

Furthermore, the measurement data for which the adjustment order is designated in the order designation field, the measurement data for which display is designated as necessary, or both of them may be included as a part of the operation control data. Alternatively, the image data of the measurement data may be included as a part of the operation control data.

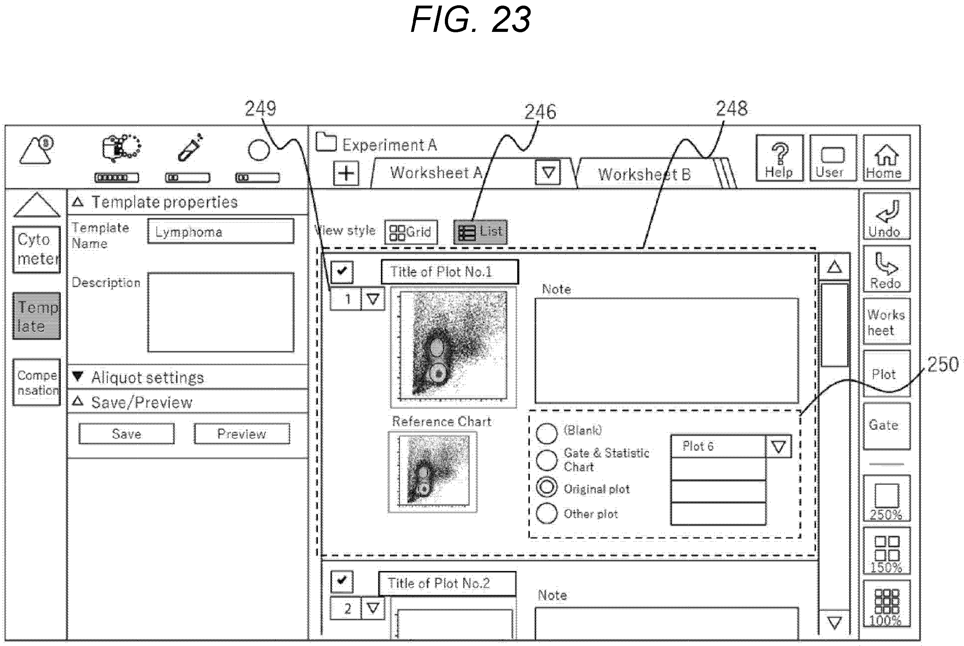

In accordance with the selecting the list display button 246 by the user, as shown in Fig. 23, the information processing device 102 displays one or more operator-oriented instruction data input areas 248 including one piece of measurement data and various instruction data input fields related to the measurement data.

An order designation field 249 for designating the adjustment order in the second display mode of the measurement data included in each area may be displayed in each operator-oriented instruction data input area. The order designation field is similar to that described above.

The operator-oriented instruction data input area includes a memo input field (Note field) for inputting a memo (particularly, an instruction memo) directed to the operator and/or a selection area 250 for selecting data to be referred to by the operator.

The memo input in the memo input field is displayed in association with the measurement data when the measurement data is displayed in the second display mode. As a result, the second user can confirm the instruction from the first user.

The first user inputs a memo directed to the second user in a memo input field. As a result, the memo data is generated. The generated data may be included in the operation control data, and in particular, may be included as a part of the "(c) operator-oriented instruction data directed to an operator operating the processing device in the second mode". The memo data display setting data is useful for accurately notifying the user in the second display mode of an instruction of the user in the first display mode.

In a case where measurement data to be referred to (hereinafter also referred to as reference measurement data) is selected in the selection area, when the measurement data is displayed in the second display mode, the reference measurement data is displayed in association with the measurement data. As a result, the second user can adjust the sorting condition with reference to the reference measurement data.

The selection area may include, for example, one, two, three, or all four of "no data to be referred to", "gate list", "ideal measurement data", and "other measurement data (plot data)" as options.

The first user selects reference measurement data in the selection area. As a result, the reference measurement data is generated. The reference measurement data may be included as part of the operation control data, and in particular, may be included as a part of the "(c) operator-oriented instruction data directed to an operator operating the processing device in the second mode". The reference measurement data is useful for adjusting processing conditions in the sorting processing in the second display mode.

In each operator-oriented instruction data input area, data to be referred to (Reference Chart field) selected in the selection area may be displayed. Furthermore, in the selection area, a list box (in the drawing, a box in which Plot 6 is displayed is the list box) for selecting data to be referred to (particularly, ideal measurement data or other measurement data) selected in the selection field may be displayed.

Furthermore, the data that can be input in the operator-oriented instruction data input area is not limited to those described above. For example, the operator-oriented instruction data input area may include an area for specifying a gate operation executable by the second user. For example, examples of the gate operation include movement of the position of the gate, change in the size of the gate (enlargement or reduction of the gate), and change in the shape of the gate. As a more specific example of change in the shape of the gate, a gate tilt change; a gate diameter or axis change (for example, change in a circular gate diameter change or an elliptical gate major axis or/and a length of a minor axis); change in the gate diameter or axis ratio (for example, change in the circular gate to an elliptical gate or vice versa, or change in the ratio of the major and minor axis lengths of an elliptical gate); and change in the eccentricity or flatness of the gate can be mentioned. For example, when the first user specifies the gate operation in the area, the information processing unit 102 generates the gate operation restriction data. In the second display mode, the information processing device 102 restricts a part of the gate operation by the second user on the basis of the gate operation restriction data. Therefore, it can prevent unnecessary gate adjustment from being performed in the second display mode.

Note that, in a case where the area for specifying the gate operation is not displayed, the information processing unit 102 may have gate operation restriction data in advance, and for example, the gate operation restriction data may be data for restricting any gate operation (for example, a change in the size of the gate and/or a change in the shape of the gate) in the second display mode. In one embodiment of the present disclosure, the gate operation restriction data may be operation restriction data for restricting the change in the shape of the gate, and for example, may be data for restricting the change in the shape of the gate but does not restrict the change in the size of the gate. Furthermore, for example, the gate operation restriction data may be inputtable in another area (such as the first display area).

As described above, the biological particle sorting device of the present disclosure displays a screen for inputting measurement data (particularly, plot data) obtained by predetermined gate setting and the operator-oriented instruction data related to the measurement data obtained by the gate setting. In the present specification, the screen is also referred to as a template generation screen. The biological particle sorting device of the present disclosure is designed to be able to input operation control data via the template generation screen, and therefore, improves convenience for the first user to generate instruction data for the second user. Furthermore, the biological particle sorting device of the present disclosure displays a processing execution operation screen for a second user in a second display mode to be described later on the basis of the operation control data. By displaying the processing execution operation screen based on the operation control data, the second user can easily perform adjustment or operation when performing sorting.

Furthermore, as shown in the drawing, an area 244 including a button for saving or previewing the generated operation control data is displayed in the setting information display area. In the area, a save button (Save button) for saving the operation control data and a preview button (Preview button) for previewing a screen (particularly, a screen displayed in the second display mode) generated on the basis of the operation control data are displayed. The information processing unit 102 executes each function described above in accordance with the selection of each button.

### (Closing Process S105)

For example, in accordance with the selection of the Closing button by the user after the sorting processing is completed in the Sort step, the biological particle sorting device 100 changes the color of the button so as to indicate a state where the Closing button is selected in the first display area (particularly, the procedure button area).

In accordance with the selecting the Closing button by the user, the biological particle sorting device 100 displays a screen for ending the setting of the sorting processing condition or a screen for ending the first display mode in the setting information display area. A button for closing the opened experiment file, a logout button for ending the first display mode, and the like may be displayed on these screens.

### (Cytometer Process)

The processing in the first display mode may optionally include a cytometer process. The cytometer process is a process for adjusting the setting of the device.

For example, in accordance with the selecting the Cytometer button by the user at an arbitrary timing of the processing in the first display mode, the biological particle sorting device 100 changes the color of the button so as to indicate a state where the Cytometer button is selected in the first display area (particularly, the procedure button area). In accordance with the selecting the Cytometer button by the user, the biological particle sorting device 100 can display a screen for adjusting the settings of the device in the setting information display area. The biological particle sorting device 100 may be designed to receive adjustment of device settings via the screen.

### (Correction Process)

The processing in the first display mode may optionally include a correction process. The correction process is a process for adjusting the fluorescence correction condition.

For example, in accordance with the selecting the Compensation button by the user at an arbitrary timing of the processing in the first display mode, the biological particle sorting device 100 changes the color of the button so as to indicate a state where the Compensation button is selected in the first display area (particularly, the procedure button area). In accordance with the selecting the Compensation button by the user, the biological particle sorting device 100 can display a screen for adjusting the settings of the fluorescence correction in the setting information display area. The biological particle sorting device 100 may be designed to receive adjustment of fluorescence correction setting via the screen.

Furthermore, although it has been described above that each step is executed in the order of arrangement in the procedure button area, the order of executing each step may be freely selected by the user. For example, the step may return to any previous step after completion of a certain step, such as returning to the Acquisition step again after the Sort step. Furthermore, the step may proceed to another step by skipping one or more steps such as proceeding to the Template step after the Analysis step. Furthermore, one or more steps may be omitted, such as omitting the Compensation step.

The first user executes the processing in each step as described above, whereby the operation control data is input. The information processing unit 102 stores the input operation control data. After the saving, the biological particle sorting device 100 may terminate the operation in the first display mode.

### (Operation Control Data)

The operation control data input in the step group described above is summarized as follows, for example.

As described above, the operation control data may include one or more of
(a) processing condition data adopted by the processing device that executes the processing in the second mode (hereinafter, also referred to as "data (a)"),
(b) display control data related to display on the processing execution operation screen in the second mode (hereinafter, also referred to as "data (b)"), and
(c) operator-oriented instruction data directed to an operator operating the processing device in the second mode (hereinafter, also referred to as "data (c)").

For example, the operation control data includes at least the data (a), more preferably the data (a) and the data (b), or the data (a) and the data (c).

Alternatively, the operational control data includes at least the data (b), more preferably the data (b) and the data (a) or the data (b) and the data (c).

Alternatively, the operational control data includes at least the data (c), more preferably the data (c) and the data (a) or the data (c) and the data (b).

In a preferred embodiment, the operation control data may include the data (a), the data (b), and the data (c) .

With the operation control data including such data, it is possible to generate a more appropriate processing execution operation screen for the user who uses the device in the second display mode.

The data (a) is processing condition data adopted in the sorting processing by the biological particle sorting device 100 in the second display mode. The data (a) may include, for example, instruction data directed to the biological particle sorting device, such as gate setting.

The data (a) includes data for specifying the biological particles to be sorted in the second display mode, and more specifically includes gate information for specifying the biological particles sorted in the sorting processing. The data may be, for example, data specifying a range of optical data generated from the biological particles to be sorted. For example, the processing condition data may include data defining a range of the biological particles to be sorted on one or more pieces of plot data. The processing condition data may be appropriately set by a person skilled in the art, and may be appropriately set by a user (for example, a researcher or a developer) who uses the biological particle sorting device 100 in the first display mode.

The data (a) may include data related to device settings adopted by the biological particle sorting device 100 in the sorting processing. For example, data related to setting of the light irradiation unit, the detection unit, and the sorting unit may be included. The data related to the setting of the light irradiation unit may be data specifying the type of light to be emitted. The data related to the setting of the detection unit may include data related to the operation setting of the detector and/or data related to the identification of the detector to be operated. The data related to the setting of the sorting unit may include data related to a pressure and/or a charge applied for the sorting operation, or data related to a voltage applied for the sorting operation.

The data (a) may include one or more pieces of the data described above which may be included (or may be handled) as the data (a). For example, the data (a) may include any one or more of setting data related to the gain and/or the threshold, setting data related to the correction matrix, correction matrix data after the adjustment, the gate information, and the timing setting data.

The data (b) is display control data related to display on the processing execution operation screen displayed in the second display mode. The data (b) may include, for example, data related to control of display of the plot data displayed in the second display mode, and more specifically, may include one or more of data related to a display position of the plot data, data related to an adjustment order of the plot data, and data related to specification of the plot data to be displayed.

The data (b) may include one or more pieces of the data described above which may be included (or may be handled) as the data (b). For example, the data (b) may include any one or more of the worksheet setting data, the plot setting data, the gate setting data, the gate operation restriction data, and the measurement data display setting data.

Particularly preferably, the data (b) includes data related to operation restriction of the plot data displayed in the second display mode. The data related to the operation restriction may include data related to prohibition of change of the plot data itself. For example, the change of the axis of the plot and/or the change of the display range of the plot may be prohibited by the data related to the prohibition. Therefore, it is possible to prevent the sorting processing intended by the user in the first display mode from not being executed due to the user in the second display mode changing the plot data.

The data (c) is operator-oriented instruction data directed to an operator who operates the biological particle sorting device in the second display mode. The instruction of the user in the first display mode can be transmitted to the user in the second display mode by the operator-oriented instruction data.

For example, the data (c) may be input for each processing step in the second display mode. As a result, it is possible to notify the user in the second display mode of the work to be executed in each processing step.

The data (c) may be input for every piece of plot data, and more specifically, may be a gate adjustment instruction input for every piece of plot data. The gate adjustment instruction enables appropriate gate setting for each sample.

The data (c) may include reference data related to confirmation and/or adjustment of the processing condition data. The reference data may include, for example, (c1) specifying data for specifying plot data changed by the adjustment, (c2) confirmation graph data to be referred to by the operator, (c3) statistical data related to each gate, and (c4) image data associated with the processing condition data.

The specifying data (c1) is, for example, data for associating plot data to be adjusted with plot data changed in accordance with the adjustment. In accordance with adjustment of the plot data to be adjusted, the biological particle sorting device 100 (particularly, the information processing unit) can specify plot data to be changed in accordance with the adjustment on the basis of the specifying data and adjust the plot data. Therefore, the biological particle sorting device 100 (particularly, the information processing unit) can execute plot data adjustment in real time according to the adjustment, for example, in the second display mode.

The confirmation graph data (c2) is, for example, reference plot data to be referred to when the operator adjusts a gate set to certain plot data in the second display mode. The biological particle sorting device 100 (particularly, the information processing unit) displays the reference plot data, so that the operator can confirm whether the sorted particles and data are as desired, particularly in the second display mode.

The statistical data related to each gate (c3) is, for example, data related to the number and/or ratio of events included in each gate. The biological particle sorting device 100 (particularly, the information processing unit) displays the statistical data, so that the operator can confirm whether the sorted particles and data are as desired, particularly in the second display mode.

The image data associated with the processing condition data (c4) may be, for example, data measured by another device or image data generated or processed as ideal data. Furthermore, the image data may be image data for indicating to the operator a countermeasure when the abnormality data is acquired, and may be, for example, an image indicating which part of the biological particle sorting device 100 should be confirmed and/or operated. Furthermore, the image data associated with the processing condition data may be image data for giving an instruction to the operator. With such image data, the biological particle sorting device 100 can give an instruction to the operator, in particular, in the second display mode.

The data (c) may include one or more pieces of the data described above which may be included as (or may be handled) as data (c). For example, the data (c) may include any one or more of the plot data, the template property data, the aliquot acquisition instruction data, the memo data, and the reference measurement data.

### (2-3) Second Display Mode

In the second display mode, the biological particle sorting device 100 displays a processing execution operation screen. The processing execution operation screen is a screen for executing processing of sorting the predetermined biological particles. At least a part of the processing execution operation screen may be generated on the basis of operation control data input in the first display mode.

Preferably, the biological particle sorting device 100 controls the display of the processing execution operation screen in the second display mode on the basis of the operation control data. Therefore, the intention of the first user regarding the sorting processing is reflected in the sorting operation performed by the second user.

Preferably, in the second display mode, the operator can adjust the processing condition data according to the operator-oriented instruction data. In the sorting processing of the biological particles, fine adjustment is often required for each sample. Since the biological particle sorting device 100 can perform the above-described adjustment, the fine adjustment can be performed.

Preferably, adjustment of at least a part of the gate information is limited in the second display mode. The sorting processing condition set by the first user often includes a condition that should not be adjusted by the second user. Therefore, since the adjustment can be restricted in this manner, it is possible to prevent an undesirable processing condition adjustment from being performed by the second user.

### (2-3-1) Example Configuration of Screen Displayed in Second Display Mode

### (Processing Execution Operation Screen)

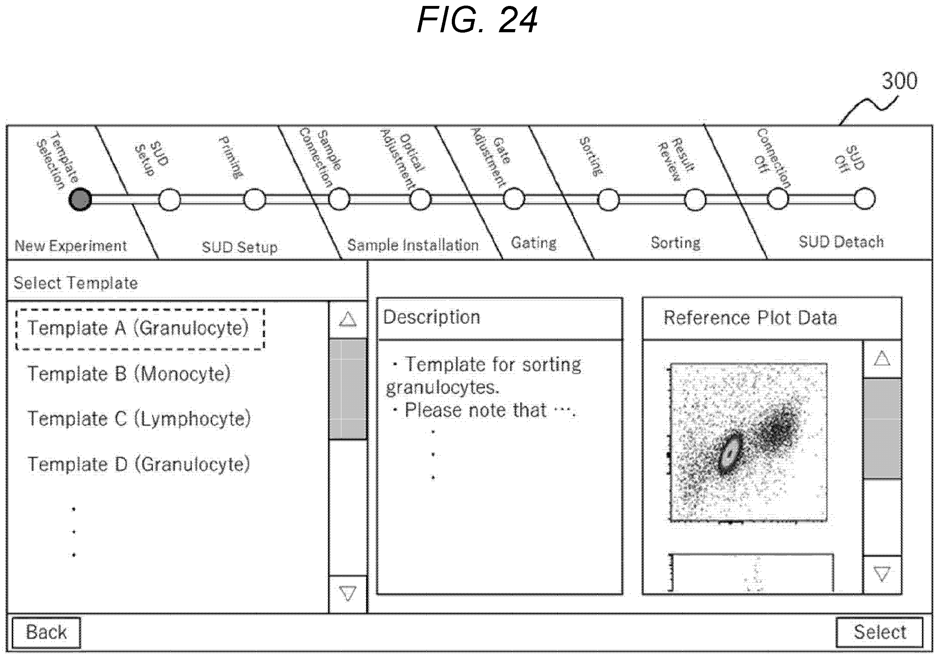

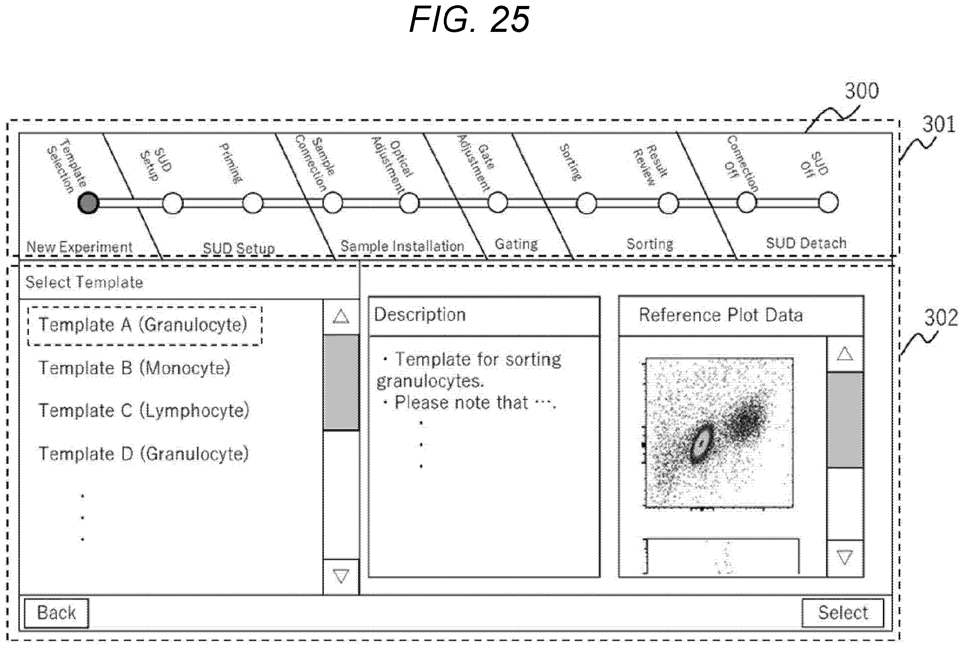

Fig. 24 illustrates an example of the processing execution operation screen displayed on the display unit 101 by the biological particle sorting device 100 (particularly, the information processing unit 102) in the second display mode. As shown in Fig. 25, the processing execution operation screen 300 shown in the 24 includes a third display area (also referred to as a processing operation flow display area) 301 for displaying the operation flow of the sorting processing, and a fourth display area (also referred to as an operation content display area) 302 for displaying the operation content in each process included in the processing operation flow.

As shown in the drawing, the third display area may be arranged on an upper side of the processing execution operation screen, and the fourth display area may be arranged on a lower side of the processing execution operation screen.

Alternatively, the third display area may be arranged on a lower side of the processing execution operation screen, and the fourth display area may also be arranged on an upper side of the processing execution operation screen.

On the processing condition setting screen in the first display mode, the two areas (the first display area and the second display area) are displayed separately on the left and right sides. On the other hand, in the second display mode, as described above, the two areas (the third display area and the fourth display area) are displayed separately on the upper and lower sides. As described above, in the first display mode, the two main areas are arranged separately on the left and right sides, and in the second display mode, the two main areas are arranged separately on the upper and lower sides, whereby the user can clearly distinguish the operation modes of the biological particle sorting device.

Furthermore, it is assumed that the second user has less knowledge about the processing conditions of the biological particle sorting device than the first user. In addition, in the second display mode, in order to prevent confusion in the sorting operation, only information necessary for the sorting operation is preferably displayed on the screen. Furthermore, the information is preferably displayed in a larger size in order to prevent an erroneous sorting operation. In the second display mode, only necessary information can be displayed in a larger size by being divided into upper and lower portions as described above, which is useful for preventing an erroneous sorting operation.

The arrangement of the third display area and the fourth display area may be changeable. For example, the biological particle sorting device may be designed to change the screen from a state where the former area is arranged on the upper side and the latter area is arranged on the lower side to a state where the former area is arranged on the lower side and the latter area is arranged on the upper side. Furthermore, the screen may be designed to be changeable so as to be opposite thereto.

### (Third Display Area)

In the third display area 301, steps (and marks indicating the steps) executed in the second display mode are arranged side by side from left to right. In addition, it is indicated by a mark (filled circle mark in gray) indicating the processing being executed. As described above, in the present disclosure, steps executed in the second display mode are shown in the third display area displayed in the second display mode, but the steps may be arranged in one direction. Furthermore, in the third display area, a mark indicating a step being executed may be displayed at the position of the step.

### (Fourth Display Area)

The contents displayed in the fourth display area 302 may be changed for each step. The content includes, for example, explanatory data related to an operation to be executed by the second user in each step and/or data indicating a status of the operation. At least a part of the content may be generated on the basis of the operation control data input in the first display mode.

### (2-3-2) Example of Processing in Second Display Mode

### (Operation Starting Processing)

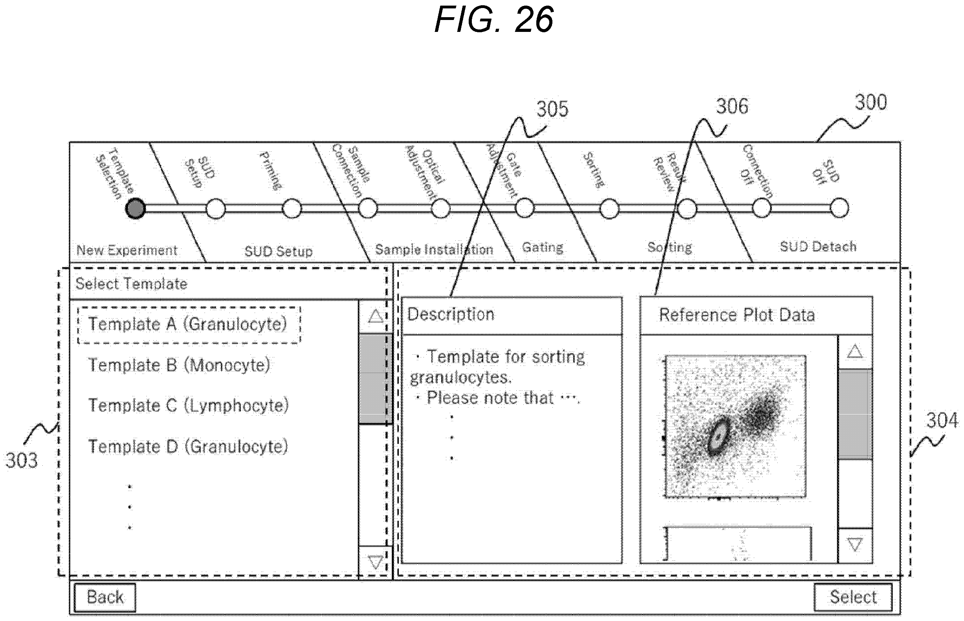

When the processing in the second display mode is started, the biological particle sorting device 100 (particularly, the information processing unit 102) causes the display unit 101 to display a processing execution operation screen 300 shown in Fig. 26.

In the drawing, the operation starting processing is displayed as "New Experiment". The operation starting processing includes a template selection process (displayed as "Template selection" in the drawing). In the third display area, the information processing unit 102 displays an instruction mark (filled circle mark in gray) indicating processing executed by the biological particle sorting device 100 at the position of the template selection process. As described above, the biological particle sorting device 100 displays the instruction mark at a position indicating the process being executed among the series of processes in the second display mode.

Furthermore, the information processing unit 102 displays a screen related to the operation content in the template selection process in the fourth display area. For example, as shown in the drawing, a template selection field 303 and a template description field 304 for describing an outline of the selected template are displayed in the fourth display area.

The information processing unit 102 displays a list of selectable templates in the template selection field. In this field, for example, a template name may be displayed on the basis of template property data, and in particular, a template name input to the template property area in the Template step in the first display mode is displayed. In the drawing, "Template A (Granulocyte) " is selected.

The information processing unit 102 displays, in the template description field, a detailed description field 305 (displayed as Description in the drawing) related to the selected template and a reference data field 306 (displayed as Reference Plot Data) displaying an example of measurement data (plot data) obtained in a case where the sorting processing is executed according to the selected template.

In the detailed description field, a description regarding the selected template is displayed. In this field, for example, a description regarding the template input in the template property area in the Template step in the first display mode may be displayed. That is, the information processing unit 102 displays a description regarding the template in the detailed description field on the basis of the template property data. For example, as shown in the drawing, description such as "template for sorting granulocytes" is displayed in the detailed description field as the description regarding Template A.

The information processing unit 102 displays an example of measurement data (plot data) obtained in a case where the sorting processing is executed according to the selected template in the reference data field. An example of the measurement data may be, for example, measurement data in which an adjustment order is designated in the Template step of the first display mode, in particular, image data. In this manner, the information processing unit 102 displays the measurement data in the reference data field on the basis of the operation control data, in particular, on the basis of the image data included in the operation control data. For example, as shown in the drawing, a plurality of pieces of plot data may be displayed in the reference data field.

The biological particle sorting device 100 advances the processing to the next process in accordance with selection of the Select button arranged at the lower right of the processing execution operation screen. In accordance with the selection of the Back button arranged at the lower right of the processing execution operation screen, the processing returns to the previous process. These buttons may also be displayed on other screens. Note that the characters in each button may be appropriately changed to other characters or marks.

### (Setup Processing)

In accordance with the template being selected in the operation starting processing, the biological particle sorting device advances the processing to setup processing. The setup processing may include, for example, an attachment process of attaching the microchip for biological particle sorting to the biological particle sorting device (displayed as "SUD setup" in Fig. 24) and a priming process of priming the inside of the flow channel of the microchip (displayed as "Priming" in the drawing). Note that in the drawing, the SUD is a name of a consumable kit (particularly, a sterilized kit) including the microchip, and other names may be described.

The process included in the setup processing and the screen displayed in each process may be appropriately changed according to the type of the biological particle sorting device and the type of the microchip to be attached.

### (Sample Introduction Processing)

After completion of the setup processing, the biological particle sorting device advances the processing to sample introduction processing. The sample introduction processing may include, for example, a sample connection process (displayed as "Sample Connection" in Fig. 24) of connecting a container containing a biological sample to be subjected to the sorting processing to the biological particle sorting device, and an optical adjustment step (displayed as "Optical Adjustment" in Fig. 24) of performing optical adjustment of the biological particle sorting device by performing a sorting operation using a part of the biological sample.

The process included in the sample introduction processing and the screen displayed in each process may be appropriately changed according to the type of the biological particle sorting device.

The biological particle sorting device may execute these processes on the basis of the operation control data (particularly, on the basis of processing condition data) .

### (Gating Processing)

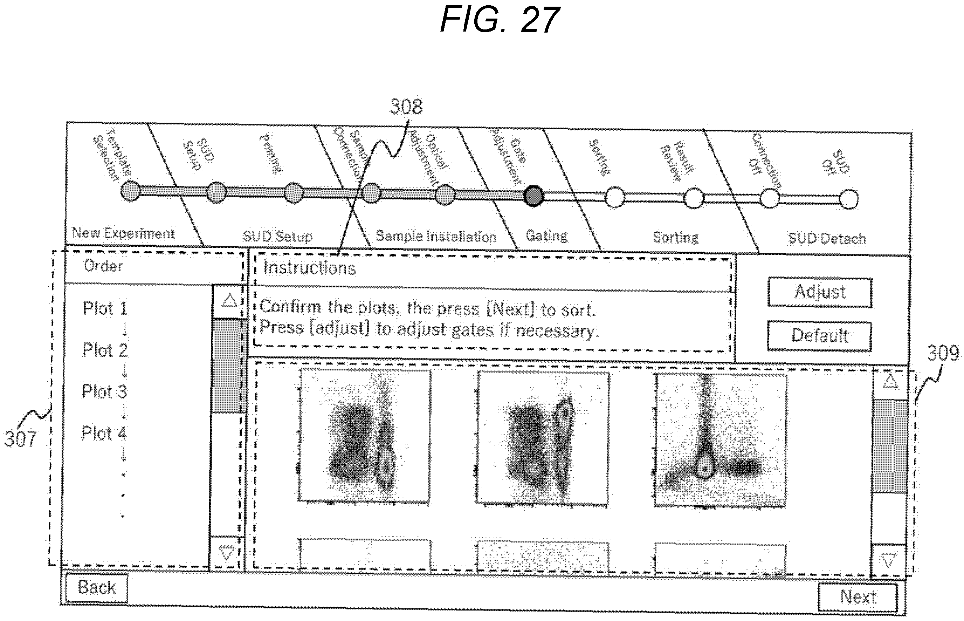

After completion of the sample introduction processing, the biological particle sorting device advances the processing to gating processing. As shown in Fig. 27, the gating processing includes, for example, a gate adjustment process (displayed as "Gate Adjustment" in Fig. 27) of adjusting the gate set for each piece of plot data in the first display mode with respect to the measurement data.

In the gate adjustment process, the biological particle sorting device 100 may perform light irradiation and detection on a part of the sample to acquire plot data. The acquisition of the plot data is executed in accordance with the processing condition data input in the first display mode.

In the gate adjustment process, as shown in Fig. 27, the biological particle sorting device 100 displays an instruction mark indicating the processing executed by the biological particle sorting device 100 at the position of the gate adjustment process in the third display area. In this manner, the second user can grasp the work situation by moving the instruction mark to the position of each process.

Furthermore, in the third display area, completed processing may be displayed differently from uncompleted processing. For example, in Fig. 27, the completed processing is displayed in gray, and the uncompleted processing is displayed in white.

Furthermore, the information processing unit 102 displays a screen related to the operation content in the gate adjustment process in the fourth display area. For example, as shown in the drawing, the fourth display area may include an adjustment order display field 307 (displayed as "Order" in the drawing), an instruction field 308 to the second user, and a data display field 309.

As shown in the drawing, the information processing unit 102 displays a list of plot data to be adjusted in the adjustment order display field on the basis of the operation control data (particularly, the measurement data display setting data, and more particularly, the data related to the adjustment order) input in the first display mode. In the list, the names of the plot data may be displayed according to the adjustment order.

As shown in drawing, the information processing unit 102 displays an instruction to the second user in an instruction field for the second user on the basis of the operation control data (particularly, the operator-oriented instruction data) input in the first display mode.

As shown in the drawing, the information processing unit 102 displays a plot data group generated by executing the sorting processing on the sample in the data display field. The information processing unit 102 may specify a plot data group to be displayed on the basis of the operation control data (particularly, display control data, and more particularly, in particular, measurement data display setting data) input in the first display mode. The plot data group may include, for example, one or more pieces of plot data corresponding to the list of plot data to be adjusted. The plot data group may further include one or more pieces of plot data not to be adjusted.

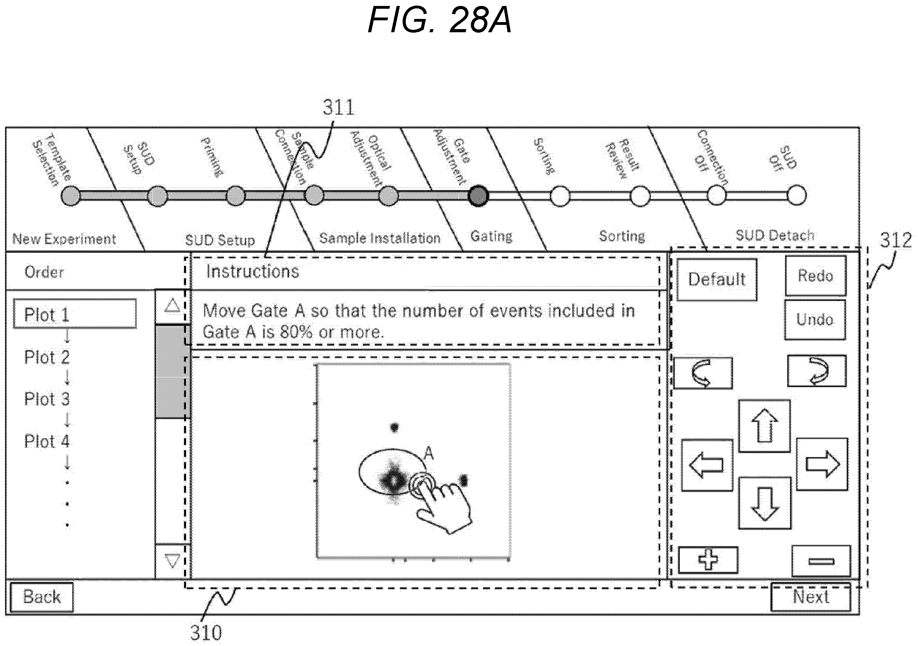

The second user confirms the plot data group according to the instruction displayed in the instruction field. Next, the second user adjusts the gates in the one or more pieces of plot data according to the adjustment order. For the adjustment, the second user selects plot data to be adjusted first from the plot data list displayed in the adjustment order display field. The selected state is shown in Fig. 28A.

In accordance with the selection of the plot 1 that is the plot data to be adjusted first by the second user, the information processing unit 102 displays the plot data of the plot 1 in the data display field 310 as shown in the drawing. In the plot data, a gate A set according to the processing condition data is also displayed.

In response to the selection of the plot 1 that is the plot data to be adjusted first by the second user, the information processing unit 102 displays an instruction related to the gate adjustment of the plot 1 in the instruction field 311 as shown in the drawing. The biological particle sorting device 100 may display the instruction on the basis of the operation control data (particularly, operator-oriented instruction data) input in the first display mode, and in particular, displays a memo (particularly, an instruction memo) input in the memo input field. The second user can accurately execute the gate adjustment by referring to the instruction field.

Furthermore, in response to selecting the plot 1 which is the plot data to be adjusted first by the second user, the biological particle sorting device 100 may display a gate operation button field 312 next to the data display field.

The gate operation button field includes a "Default" button for returning the gate to the state before adjustment by the second user, a "Redo" button for redoing the operation performed on the gate or performing the canceled operation again, and an "Undo" button for canceling the operation performed on the gate. Furthermore, the gate operation button field may include a button group for operating the gate position and/or a button group for changing the size or shape of the gate.

The biological particle sorting device 100 may be designed such that a part of the gate operation is restricted in the second display mode. For example, one or both of the size and the shape of the gate may by designed so that it cannot be changed. The information processing unit 102 may limit the operation on the basis of the operation control data. In one embodiment, the information processing unit 102 restricts the change of the shape of the gate but permits the change of the size of the gate on the basis of the operation control data (particularly, display control data, and more particularly, gate operation restriction data). For example, the information processing unit 102 can change the size of the gate A in accordance with the second user touching and dragging the gate A. For example, the information processing unit 102 can change the size of the gate A in accordance with dragging in a state where the second user touches a specific position (for example, a size change mark displayed in the gate A or displayed on a line defining the gate A, a line defining the gate A, or the like).

Furthermore, the information processing unit 102 may permit a change in the position of the gate on the basis of the operation control data. For example, the information processing unit 102 can change the position of the gate A in accordance with the second user touching and dragging the gate A. For example, the information processing unit 102 may change the size of the gate A in accordance with dragging a specific position (for example, a size change mark displayed in the gate A or displayed on the line defining the gate A, or a line defining the gate A, or the like) while being touched by the second user.

After the gate adjustment of the plot 1 is completed, the second user selects a plot to be adjusted next from among the plot groups listed in the adjustment order display field, and then performs gate adjustment for the selected plot. Similarly to the plot 1, the gate adjustment may be executed according to an operator-oriented instruction included in the operation control data.

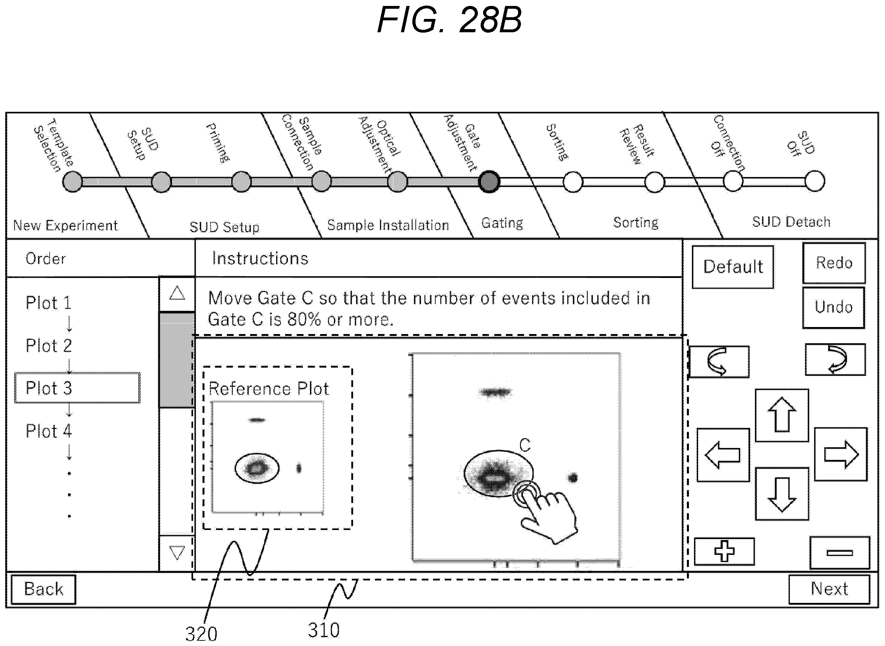

An example of a screen on which other plot data to be adjusted is selected is shown in Fig. 28B. In the drawing, the plot 3 is selected as plot data to be adjusted.

In accordance with the selection of the plot 3 by the second user, the information processing unit 102 displays the plot data of the plot 3 in the data display field 310 as shown in the drawing. In the plot data, a gate C set according to the processing condition data is also displayed.

Furthermore, as shown in the drawing, the information processing unit 102 may display the confirmation graph data (Reference Plot) to be referred to for adjustment of the gate C in the data display field 310. The information processing unit 102 can display the confirmation graph data on the basis of the operation control data (particularly, the operator-oriented instruction data, and more particularly, reference data). As shown in the drawing, the confirmation graph data may include an image of the plot data acquired in the first display mode and a gate set for the plot data by the user in the first display mode. The output of the confirmation graph data to the screen is useful for the user in the second display mode to accurately execute the gate adjustment in the plot data.

In response to completion of all the gate adjustment of one or more plots to be adjusted, the biological particle sorting device 100 advances the processing to the sorting processing.

### (Sorting Processing)

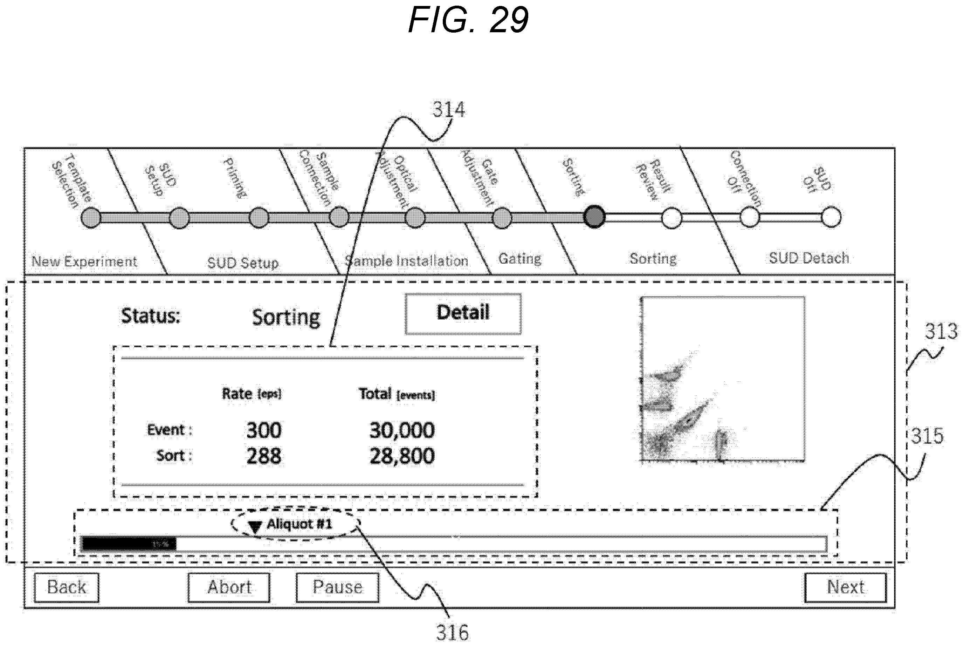

After completion of the gate adjustment processing, the biological particle sorting device advances the processing to sorting processing. As shown in Fig. 29, the sorting processing may include, for example, a sorting process (displayed as "Sorting" in the drawing) of executing sorting of the biological particles and a result confirmation process (displayed as "Result Review") of confirming a result of the sorting.

Furthermore, the biological particle sorting device may execute an aliquot acquisition process for confirming whether the biological particles being sorted are the target biological particles in the middle of the sorting process on the basis of the operation control data (particularly, aliquot setting data). By the confirmation in the aliquot acquisition step, the second user can confirm whether the target biological particle is sorted, and in a case where the target biological particles are not sorted, it is possible to prevent the valuable sample from being wastefully consumed by interruption of the sorting processing or adjustment of the processing condition. For the confirmation, analysis by another biological particles analyzer may be performed as described above.

In the sorting process, as shown in the drawing, the information processing unit 102 displays a screen 313 indicating a sorting status in the fourth display area. As shown in the drawing, an area 314 displaying the number of events and/or the event acquisition speed (eps) and the number of sorted particles and/or the sorting speed (eps) may be displayed on the screen.

The information processing unit 102 may display an image 315 (in the drawing, a bar is displayed as the image) indicating the progress status of the sorting operation on the screen. An image 316 indicating the timing at which the aliquot acquisition process is executed may be attached to the bar. Therefore, the second user can know that the timing of executing the aliquot acquisition process comes, and can perform, for example, preparation work and other work by the timing, and convenience for the second user can be improved. The information processing unit 102 may display an image indicating the timing on the basis of the control data input in the first display mode, in particular, on the basis of the aliquot setting data.

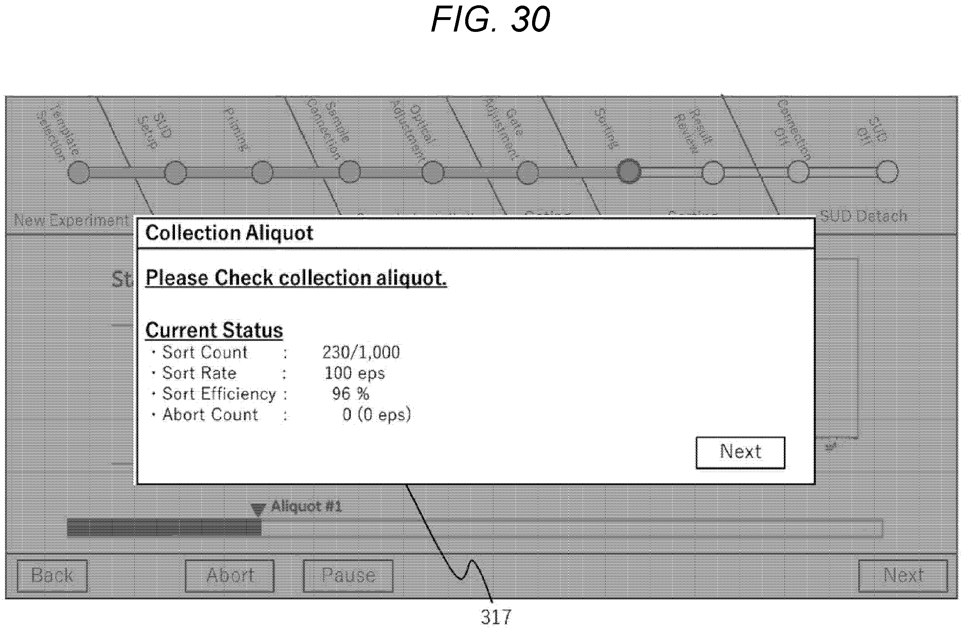

When the timing to execute the aliquot acquisition process arrives, the biological particle sorting device 100 temporarily stops the sorting processing and displays a window 317 shown in Fig. 30, for example. As shown in the drawing, the confirmation method for the aliquot and/or the current sorting status may be displayed on the window. Furthermore, a button for proceeding with the processing next is also displayed on the window.

In accordance with the selection of the button, the information processing unit 102 displays a screen prompting the second user to select whether to continue the sorting processing. The screen includes a continue button for causing the information processing unit 102 to continue the sorting processing and a stop button for stopping the sorting processing. In accordance with selecting the proceed button by the second user, the information processing unit 102 continues the sorting processing. In accordance with selecting the stop button by the second user, the biological particle sorting device 100 stops the sorting processing. Note that the screen may include a readjustment button for performing gate adjustment again. In accordance with the selection of the readjustment button by the second user, the biological particle sorting device 100 returns the processing to the gate adjustment processing.

In the result confirmation process, the biological particle sorting device 100 displays a screen indicating a sorting result in the fourth display area. The number of events and/or the sorted number may be displayed on the screen.

### (End Processing)

After completion of the sorting processing, the biological particle sorting device 100 advances the processing to end processing. As shown in Fig. 24, the end processing may include, for example, a collecting container removing process (displayed as "Collection Off" in the drawing) of collecting a container containing the sorted biological particles from the biological particle sorting device 100, and a chip removing process (displayed as "SUD Off") of removing a microchip used for sorting from the biological particle sorting device 100. The screen displayed in the fourth display area for these processes may be appropriately selected by those skilled in the art.

The sorting processing in the second display mode is executed by the above processing.

### (3) Modified Example

In (1) and (2) described above, the biological particle sorting device that executes the sorting processing on the biological particles contained in the biological sample flowing through the flow channel (particularly, the biological particles flowing side by side in the flow channel) has been described. However, the present disclosure may be applied to a biological particle sorting device that executes the sorting processing on a biological particle group existing two-dimensionally or three-dimensionally. That is, the biological particle sorting device of the present disclosure may be designed to execute sorting processing on a biological particle group existing two-dimensionally or three-dimensionally. Examples of the biological particle sorting device configured as described above include a biological particle sorting device that executes sorting processing on a biological particle group existing in a well arranged two-dimensionally or three-dimensionally, a biological particle sorting device that executes sorting processing on a biological particle group existing two-dimensionally or three-dimensionally on an arbitrary support (for example, a cell culture surface or a cell immobilization surface), and a biological particle sorting device that executes biological particle sorting processing on a biological particle group (for example, a biological tissue) forming a three-dimensional structure.

As an example of the biological particle sorting device that executes sorting processing on a biological particle group existing in the well, a biological particle sorting device that executes biological particle sorting processing using the particle capturing chip described in JP 2020-174598 A can be mentioned.

As an example of the biological particle sorting device that executes sorting processing on a biological particle group existing two-dimensionally on the arbitrary surface, a biological particle sorting device that sorts a specific biological particle from a biological particle (cell) group immobilized via a linker (for example, a photodegradable linker) decomposable on a certain surface can be mentioned.

These devices may be designed to acquire fluorescence signals or fluorescence images of the biological particles using, for example, an optical detector including a microscope or the like, and identify the biological particles to be sorted on the basis of the acquired fluorescence signals or fluorescence images.

These devices may be designed to remove only the biological particles identified in this manner from the well, or may be designed to release the immobilization of only the biological particles identified in this manner (for example, to cleave a linker that immobilizes the biological particles).

These devices may be designed to include the first display mode and the second display mode described in (1) and (2) described above. For example, in the first display mode, operation control data for specifying the biological particles to be sorted is input. Then, in the second display mode, a processing execution operation screen is displayed on the basis of the operation control data.

### 3. Second Embodiment of the Present Disclosure (Biological Particle Sorting System)

The present disclosure also provides a biological particle sorting system including a first information processing device that operates in a first display mode for receiving an input of operation control data related to biological particle sorting processing and a second information processing device that operates in a second display mode in which a processing execution operation screen is displayed.

The biological particle sorting system further includes a biological particle sorting device operated by the first information processing device or the second information processing device. The biological particle sorting device may have the configuration in 2. described above.

Furthermore, the present disclosure also provides the first information processing device included in the biological particle sorting system. The first information processing device may include, for example, a touch inputtable display unit that displays a processing condition setting screen that receives an input of operation control data related to the sorting processing of the biological particle-containing sample. The processing condition setting screen may be as described in 2. described above, and the description also applies to the present embodiment.

Furthermore, the present disclosure also provides the second information processing device included in the biological particle sorting system.

The second information processing device may include, for example, a touch inputtable display unit that displays a processing execution operation screen. The processing execution operation screen may be as described in 2. described above, and the description also applies to the present embodiment.

An example of a biological particle sorting system according to the present disclosure will be described with reference to Fig. 31. The drawing shows an example configuration of a biological particle sorting system of the present disclosure. A biological particle sorting system 400 shown in the drawing includes a biological particle sorting device 401 that executes biological particle sorting processing, and a plurality of information processing devices 402a to 402c configured to be able to operate the biological particle sorting device. The number of information processing devices included in the system is not limited to three as shown in the drawing, and may be one or more. The plurality of information processing devices is connected to the biological particle sorting device via, for example, a network 403.

Hereinafter, a case where the information processing device 402a shown in the drawing operates in the first display mode described above and the information processing device 402b operates in the second display mode described above will be described.

First, the information processing device 402a executes steps S10, S11, and S12 described in (2-1) of 2. described above. Then, next, the information processing device 402a executes the processing in the first display mode described in (2 -2) of 2. described above to generate operation control data.

More specifically, the information processing device 402a operates as the information processing unit 102 described in (2-2) of 2. described above. The information processing device 402a causes a display unit attached to or connected to the device to output the processing condition setting screen described in (2-2) of 2. described above, and receives an input of the operation control data via the screen. In addition, in the generation of the operation control data, sorting processing by the biological particle sorting device 401 may be performed.

The information processing device 402a transmits the generated operation control data to the biological particle sorting device 401.

Next, the information processing device 402b executes steps S10, S11, and S12 described in (2-1) of 2. described above. Next, the information processing device 402b receives the operation control data from the biological particle sorting device 401. Then, the information processing device 402b executes the processing in the second display mode described in (2-3) of 2. described above on the basis of the operation control data, and causes the biological particle sorting device 401 to execute the sorting processing.

More specifically, the information processing device 402b operates as the information processing unit 102 described in (2-3) of 2. described above. The information processing device 402b causes a display unit attached to or connected to the device to output the processing execution operation screen described in (2-3) of 2. described above, and causes the biological particle sorting device 401 to perform sorting processing via the screen.

The biological particles are sorted by a series of procedures as described above.

Note that the present disclosure can also have the following configurations.
[1] A biological particle sorting device that operates in a first display mode or a second display mode on the basis of identification information, the device including:
   a first display mode for receiving an input of operation control data related to biological particle sorting processing; and
   a second display mode in which a processing execution operation screen is displayed.
[2] The biological particle sorting device according to [1], in which,
   in the first display mode, a processing condition setting screen related to the biological particle sorting processing is displayed, and
   input of the operation control data is received via the processing condition setting screen.
[3] The biological particle sorting device according to [1] or [2], in which
   the operation control data includes one or more of:
   (a) processing condition data adopted in sorting processing by the biological particle sorting device in the second display mode;
   (b) display control data related to display on the processing execution operation screen in the second display mode; and
   (c) operator-oriented instruction data directed to an operator who operates the biological particle sorting device in the second display mode.
[4] The biological particle sorting device according to [3], in which the processing condition data includes gate information for specifying biological particles sorted in the sorting processing.
[5] The biological particle sorting device according to [4], in which
   the gate information is designed to be adjusted in the first display mode, and
   the adjusting of the gate information includes performing one or more of moving a position of a gate, enlarging or reducing the gate, and changing an inclination of the gate.
[6] The biological particle sorting device according to any one of [1] to [4], in which the biological particle sorting device controls display of the processing execution operation screen in the second display mode on the basis of the operation control data.
[7] The biological particle sorting device according to [3], in which, in the second display mode, the operator can adjust the processing condition data according to the operator-oriented instruction data.
[8] The biological particle sorting device according to [4], in which adjustment of at least a part of the gate information is restricted in the second display mode.
[9] The biological particle sorting device according to [8], in which change of a shape of the gate in the gate information is restricted.
[10] The biological particle sorting device according to any one of [3] to [7], in which the operator-oriented instruction data includes reference data related to confirmation and/or adjustment of the processing condition data.
[11] The biological particle sorting device according to [10], in which
   the reference data includes one or more of:
   specifying data for specifying plot data to be changed by the adjustment;
   confirmation graph data to be referred to by the operator;
   statistical data related to each gate; and
   image data associated with the processing condition data.
[12] The biological particle sorting device according to [3], in which the display control data includes data related to control of display of plot data displayed in the second display mode.
[13] The biological particle sorting device according to [3], in which the display control data includes adjustment order data for specifying an adjustment order of plot data in the second display mode and/or display necessity data for specifying necessity of display of the plot data in the second display mode.
[14] The biological particle sorting device according to any one of [1] to [13], in which the first display mode is designed such that aliquot acquisition instruction data related to aliquot acquisition in the sorting processing can be included in the operation control data.
[15] The biological particle sorting device according to any one of [1] to [14], in which
   the processing execution operation screen displayed in the second display mode includes:
   a third display area for displaying an operation flow of the sorting processing; and
   a fourth display area for displaying operation content in each process included in the operation flow of the processing.
[16] The biological particle sorting device according to [15], in which
   the third display area is arranged on an upper side of the processing execution operation screen, and the fourth display area is arranged on a lower side of the processing execution operation screen, or
   the third display area is arranged on a lower side of the processing execution operation screen, and the fourth display area is arranged on an upper side of the processing execution operation screen.
[17] The biological particle sorting device according to [2], in which
   the processing condition setting screen displayed in the first display mode includes:
   a first display area for displaying an operation flow for setting a processing condition of the sorting processing; and
   a second display area for displaying measurement data related to a biological particle-containing sample.
[18] The biological particle sorting device according to [17], in which
   the first display area is arranged on a left side of the processing condition setting screen, and the second display area is arranged on a right side of the processing condition setting screen, or
   the first display area is arranged on a right side of the processing condition setting screen, and the second display area is arranged on a left side of the processing condition setting screen.
[19] The biological particle sorting device according to any one of [1] to [18], in which
   the biological particle sorting device includes a display device that displays a processing execution operation screen displayed in the second display mode.
[20] An information processing device for controlling biological particle sorting processing
   that is designed to receive input of operation control data related to biological particle sorting processing via a processing condition setting screen related to the biological particle sorting processing, and transmit the operation control data to a biological particle sorting device that executes the sorting processing or an information processing device that operates the sorting device, in which
   the operation control data includes processing condition data adopted by the biological particle sorting device.
[21] An information processing device for executing biological particle sorting processing
   that is designed to receive operation control data related to biological particle sorting processing and display a processing execution operation screen generated on the basis of the operation control data, and
   cause the biological particle sorting device to execute sorting processing according to an instruction input by an operator via the processing execution operation screen, in which
   the operation control data includes gate information for specifying biological particles sorted in the processing, and
   adjustment of at least a part of the gate information is restricted in the processing execution operation screen.
[22] A biological particle sorting system including:
   a first biological particle sorting device or information processing device that operates in a first display mode for receiving an input of operation control data related to biological particle sorting processing; and
   a second biological particle sorting device or information processing device that operates in a second display mode in which a processing execution operation screen is displayed.

### REFERENCE SIGNS LIST

- 100: Biological particle sorting device
- 101: Display unit
- 102: Information processing unit

## Claims

1. A biological particle sorting device that operates in a first display mode or a second display mode on a basis of identification information, the device comprising:
a first display mode for receiving an input of operation control data related to biological particle sorting processing; and
a second display mode in which a processing execution operation screen is displayed.

2. The biological particle sorting device according to claim 1, wherein,
in the first display mode, a processing condition setting screen related to the biological particle sorting processing is displayed, and
input of the operation control data is received via the processing condition setting screen.

3. The biological particle sorting device according to claim 1, wherein
the operation control data includes one or more of:
(a) processing condition data adopted in sorting processing by the biological particle sorting device in the second display mode;
(b) display control data related to display on the processing execution operation screen in the second display mode; and
(c) operator-oriented instruction data directed to an operator who operates the biological particle sorting device in the second display mode.

4. The biological particle sorting device according to claim 3, wherein the processing condition data includes gate information for specifying biological particles sorted in the sorting processing.

5. The biological particle sorting device according to claim 4, wherein
the gate information is designed to be adjusted in the first display mode, and
the adjusting of the gate information includes performing one or more of moving a position of a gate, enlarging or reducing the gate, and changing an inclination of the gate.

6. The biological particle sorting device according to claim 1, wherein the biological particle sorting device controls display of the processing execution operation screen in the second display mode on a basis of the operation control data.

7. The biological particle sorting device according to claim 3, wherein, in the second display mode, the operator can adjust the processing condition data according to the operator-oriented instruction data.

8. The biological particle sorting device according to claim 4, wherein adjustment of at least a part of the gate information is restricted in the second display mode.

9. The biological particle sorting device according to claim 8, wherein change of a shape of the gate in the gate information is restricted.

10. The biological particle sorting device according to claim 3, wherein the operator-oriented instruction data includes reference data related to confirmation and/or adjustment of the processing condition data.

11. The biological particle sorting device according to claim 10, wherein
the reference data includes one or more of:
specifying data for specifying plot data to be changed by the adjustment;
confirmation graph data to be referred to by the operator;
statistical data related to each gate; and
image data associated with the processing condition data.

12. The biological particle sorting device according to claim 3, wherein the display control data includes data related to control of display of plot data displayed in the second display mode.

13. The biological particle sorting device according to claim 3, wherein the display control data includes adjustment order data for specifying an adjustment order of plot data in the second display mode and/or display necessity data for specifying necessity of display of the plot data in the second display mode.

14. The biological particle sorting device according to claim 1, wherein the first display mode is designed such that aliquot acquisition instruction data related to aliquot acquisition in the sorting processing can be included in the operation control data.

15. The biological particle sorting device according to claim 1, wherein
the processing execution operation screen displayed in the second display mode includes:
a third display area for displaying an operation flow of the sorting processing; and
a fourth display area for displaying operation content in each process included in the operation flow of the processing.

16. The biological particle sorting device according to claim 15, wherein
the third display area is arranged on an upper side of the processing execution operation screen, and the fourth display area is arranged on a lower side of the processing execution operation screen, or
the third display area is arranged on a lower side of the processing execution operation screen, and the fourth display area is arranged on an upper side of the processing execution operation screen.

17. The biological particle sorting device according to claim 2, wherein
the processing condition setting screen displayed in the first display mode includes:
a first display area for displaying an operation flow for setting a processing condition of the sorting processing; and
a second display area for displaying measurement data related to a biological particle-containing sample.

18. The biological particle sorting device according to claim 17, wherein
the first display area is arranged on a left side of the processing condition setting screen, and the second display area is arranged on a right side of the processing condition setting screen, or
the first display area is arranged on a right side of the processing condition setting screen, and the second display area is arranged on a left side of the processing condition setting screen.

19. The biological particle sorting device according to claim 1, wherein
the biological particle sorting device includes a display device that displays a processing execution operation screen displayed in the second display mode.

20. An information processing device for controlling biological particle sorting processing
that is designed to receive input of operation control data related to biological particle sorting processing via a processing condition setting screen related to the biological particle sorting processing, and transmit the operation control data to a biological particle sorting device that executes the sorting processing or an information processing device that operates the sorting device, wherein
the operation control data includes processing condition data adopted by the biological particle sorting device.

21. An information processing device for executing biological particle sorting processing
that is designed to receive operation control data related to biological particle sorting processing and display a processing execution operation screen generated on a basis of the operation control data, and
cause the biological particle sorting device to execute sorting processing according to an instruction input by an operator via the processing execution operation screen, wherein
the operation control data includes gate information for specifying biological particles sorted in the processing, and
adjustment of at least a part of the gate information is restricted in the processing execution operation screen.

22. A biological particle sorting system comprising:
a first biological particle sorting device or information processing device that operates in a first display mode for receiving an input of operation control data related to biological particle sorting processing; and
a second biological particle sorting device or information processing device that operates in a second display mode in which a processing execution operation screen is displayed.
